# EUROPEAN PATENT SPECIFICATION

(11) **EP 0 523 978 B1**
(45) Date of publication and mention of the grant of the patent: **12.05.1999**
(21) Application number: 92306477.8
(22) Date of filing: 15.07.1992
(51) Int. Cl.: C07F 9/655, G01N 33/543, C07H 21/00, C07F 9/24, C07F 9/146, C07F 9/141, C07F 9/09, C07D 317/20, C07D 317/36, C07C 69/96, C07C 323/11

(54) **Modified phosphorous intermediates for providing functional groups on the 5' end of oligonucleotides**
Modifizierte, Phosphor enthaltende Zwischenprodukte zur Erzeugung von funktionalen Gruppen am 5' Ende von Oligonucleotiden
Intermédiaires modifiés contenant de phosphore pour la préparation de groupes fonctionnelles à la position 5' d'oligonucléotides

(30) Priority: 15.07.1991 US 731055
(43) Date of publication of application: 20.01.1993
(73) Proprietor: LA JOLLA PHARMACEUTICAL, San Diego, California 92121 (US)
(72) Inventor: Jones, David S., San Diego, California 92127 (US); Hachmann, John P., No.128 San Diego, California 92130 (US); Conrad, Michael J., San Diego, California 92129 (US); Coutts, Stephen, Rancho Sante Fe, California 92067 (US); Livingston, Douglas Alan, No.115 San Diego, California 92122 (US)
(74) Representative: Goldin, Douglas Michael

(56) References cited:
- EP-A- 0 147 768
- EP-A- 0 354 323
- EP-A- 0 399 330
- WO-A-85/03704
- WO-A-87/02777
- DE-A- 3 937 116
- US-A- 3 225 063
- CHEMICAL & PHARMACEUTICAL BULLETIN vol. 26, no. 5, May 1978, pages 1493 - 1500; N. MITSUO ET AL: 'Preparation of Lecithin-Type of Phospholipid Analogues and Mesomorphic States'
- CHEMICAL ABSTRACTS, vol. 115, no. 17, 28 October 1991, Columbus, Ohio, US; abstract no. 183758, K.C. GUPTA ET AL: 'A general method for the synthesis of 3'-sulfhydryl and phosphate group containing oligonucleotides' page 976 ;column 2 ;

## Description

This invention is in the field of organophosphate chemistry and solid state oligonucleotide synthesis. More particularly, it concerne reactive phosphorous intermediates that may be stably attached to the 5' end of an oligonucleotide and which have an activatable moiety which, when activated, provides a functional aldehyde or sulfhydryl group that may be used to conjugate the oligonucleotide to any molecule having a free amino group.

### Background

It is necessary to provide oligonucleotides with a free functional group in order to couple the oligonucleotide to labels, ligands, solid surfaces, polymers or other molecules or surfaces.

One technique for providing oligonucleotides with a terminal functional group involves synthesizing the desired oligonucleotide by conventional solid-state automated synthesis procedures and incorporating the functional group at the 5' end of the oligonucleotide via a modified phosphoramidite.

Agrawal, S., et al., Nucl. Acids Res. (1986) 14:6227-6245, describes a modified phosphoramidite that may be introduced on the 5' end of an oligonucleotide that has an activatable group that may be activated through deprotection to provide a free amino group on the 5' terminus of the oligonucleotide. The linker (VIII on page 6236), O-(2-(9-fluorenylmethoxycarbonyl) aminoethyl) -O-(2-cyanoethyl)-N-N-diisopropyl phosphoramidite, is added to the end of the desired oligonucleotide on an automated DNA synthesizer using deoxynucleoside-2-cyanoethyl-N-N-diisopropyl phosphoramidites. The adduct is deprotected (the 9-fluorenylmethoxycarbonyl group is removed with ammonia) to provide a free amino group.

Kremsky, J.N., et al., Nucl, Acids Res. (1987) 15:2891-2909, describes a functionalized phosphoramidite (1 on page 2893) that is introduced onto the 5' end of an oligonucleotide and then modified to provide a 5' carboxy or aldehyde group that is used to immobilize the oligonucleotide.

Another functionalized phosphoramidite, O-6-(4',4"-dimethoxytriphenylmethylthio)hexyl-O-(2-cyanoethyl) -N,N-diisopropylphosphoramidite, is available commercially from Clontech Laboratories. This molecule is incorporated into oligonucleotides using conventional phosphoramidite protocols. The dimethoxytrityl-protected sulfhydryl group may be deprotected with silver nitrate to yield a free sulfhydryl at the 5' end of the oligonucleotide chain.

A principal object of the present invention is to provide novel modified phosphorous intermediates that may be employed in the various types of oligonucleotide synthesis methods and which have activatable groups that may be converted to a free aldehyde or sulfhydryl group once they have been added onto the 5' end of an oligonucleotide. The free aldehyde/ sulfhydryl group is useful for coupling or conjugating the oligonucleotide to labels, ligands, polymers or solid surfaces. These new intermediates meet the following criteria: 1) the activatable group is compatible with all steps of conventional oligonucleotide synthesis procedures; 2) the activation is effected under conditions that do not damage the oligonucleotide; 3) the coupling is effected under conditions that do not damage the oligonucleotide or the moiety to which the oligonucleotide is coupled.

### Disclosure of the Invention

The novel phosphorus-containing compounds of the invention include intermediates that are useful in the H-phosphonate, phosphotriester, phosphorchloridite and phosphoramidite methods of oligonucleotide synthesis as well as intermediates that result in 5' modifications that involve phosphodiester analogs such as methyl phosphonates, methyl phosphates, phosphorothioates and phosphoramidates.

These compounds are of the formula: 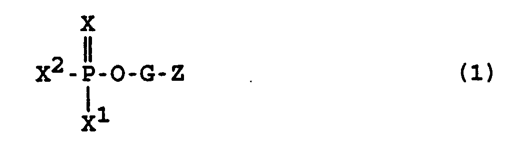 where
X is:
   (i) an oxygen or sulphur atom when X¹ represents O⁻, methyl or -OCH₃ and X² represents a hydrogen atom or RO- where R represents a protecting group;
   (ii) not present when
      (a) X¹ represents a chlorine atom and X² represents methyl or RO-; or
      (b) X² represents RO- and X¹ represents NR¹R² where each R¹ and R² individually represents C₁₋₆ alkyl, C₃₋₈ cycloalkyl or a C₆₋₂₀ aryl group or, when joined together, form with the nitrogen atom a C₄₋₇ cyclic structure with 0 or 1 oxygen or sulphur atoms;
G represents a C₁₋₂₀ hydrocarbylene group; and
Z represents a hydroxy protected vicinal diol group bound to G by one of the vicinal diol carbon atoms or a disulfide group bound to G by one of the sulfur atoms of the disulfide group;
with the proviso that G has at least 4 carbon atoms when Z represents said disulfide group.

The above compounds where X is oxygen, X¹ is O⁻, and X² is hydrogen are H-phosphonates and are employed in the H-phosphonate method of oligonucleotide synthesis (Sinha and Cook, NAR (1988) 16:2659-2669). H-phosphonates may be converted to phosphite diesters, phosphorothioates, or phosphoramidates once they are incorporated onto the 5' end of the oligonucleotide (Miller et al., NAR (1983) 11:5189-5204, Eckstein, Ann Rev Biochem (1985) 54:367-402, and Froehler and Matteucci, NAR (1988) 16:4831-4839). Correspondingly, the above compounds where X is oxygen, X¹ is O⁻ and X² is RO- are used in the phosphotriester approach to synthesizing oligonucleotides (Garegg, et al., Chemica Scripta (1985) 26:5). When X is not present and X¹ is chlorine and X² is RO-, the resulting compound is a phosphochloridite and it is used in the phosphochloridite technique for oligonucleotide synthesis (Wada et al., J Org Chem (1991) 56:1243-1250). The phosphoramidites of the above formula are preferred.

The preferred phosphoramidites of the invention may be represented by the formula: 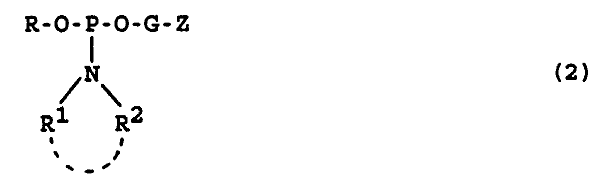
where R represents a methyl group or base-labile protecting group;
each R¹ and R² individually represents a C₁₋₆ alkyl, C₃₋₈ cycloalkyl, or a C₆₋₂₀ aryl group or, when joined together, form with the nitrogen atom C₄₋₇ cyclic structure having 0 or 1 oxygen or sulphur atoms;
G represents a C₁₋₂₀ hydrocarbylene group; and
Z represents a hydroxy-protected vicinal diol group bound to G by one of the vicinal diol carbon atoms or a disulfide group bound to G by one of the sulfur atoms of the disulfide group;
with the proviso that G has at least 4 carbon atoms when Z represents said disulfide group.

Another aspect of the invention is a 5' modified oligonucleotide of the formula: 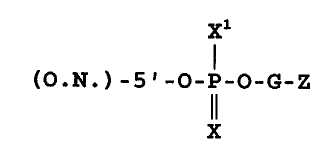 where (O.N.) represents an oligonucleotide chain;
X is an oxygen or sulphur atom;
X¹ represents O⁻, methyl, -OCH₃ or NR¹R² where each R¹ and R² individually represent a hydrogen atom or a C₁₋₆ alkyl, C₃₋₈ cycloalkyl or C₆₋₂₀ aryl group or, when joined together, form with the nitrogen atom a C₄₋₇ cyclic structure containing none or one oxygen or sulphur atoms; G is a C₁₋₂₀ hydrocarbylene group; and
Z is a hydroxy-protected vicinal diol group bound to G by one of the vicinal diol carbon atoms or a disulfide group bound to G by one of the sulfur atoms of the disulfide group.

A further aspect of the invention is the above-described modified oligonucleotides where the hydroxy protecting groups have been removed to leave free hydroxyl groups.

Yet another aspect of the invention is the above-described 5'-modified oligonucleotide in which Z represents a deprotected vicinal diol group which has been oxidized to form a terminal aldehyde group on the oligonucleotide.

Another aspect of the invention is a conjugate of the above-described oligonucleotide having a terminal aldehyde group and a free amino group-containing carrier molecule wherein the conjugate is formed by reaction between the aldehyde group and the free amino group.

A further aspect of the invention is a partially protected triol of the formula: 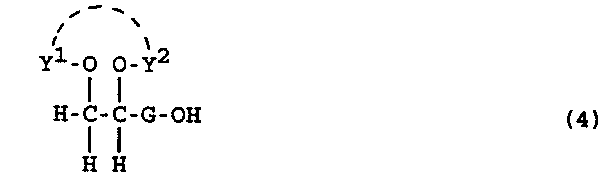 where each Y¹ and Y² individually represents a hydroxy protecting group or are together joined by a single-atom bridge, other than a -C(=O)-bridging group, to form a five-membered ring protecting group and G represents a C₂₋₂₀ hydrocarbylene group.

Preferably G is alkylene of 4 to 20 carbon atoms.

Another aspect of the invention is a disulfide of the formula

Y³-O-G-S-S-G-OH (5)

wherein Y³ is a hydroxyl protecting group and G is as described above. The two divalent groups represented by G may be the same or different. Preferably they are the same, making the disulfide symmetrical. Preferably Y³ is base stable. Preferably G is alkylene of 4 to 20 carbon atoms.

### Brief Description of the Drawings

Figures 1-3 are schematic diagrams of the synthesis schemes described in Examples 1-3.

Figures 4 and 5 are autoradiograms of the gels described in Examples 5 and 6.

### Modes for Carrying Out the Invention

As indicated above, the phosphoramidites of the invention may be represented by the formula: 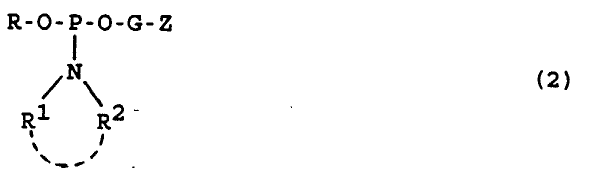
where R represents a methyl group or base-labile protecting group;
each R¹ and R² individually represents a C₁₋₆ alkyl, C₃₋₈ cycloalkyl, or a C₆₋₂₀ aryl group or, when joined together, form with the nitrogen atom C₄₋₇ cyclic structure having 0 or 1 oxygen or sulphur atoms;
G represents a C₁₋₂₀ hydrocarbylene group; and
Z represents a hydroxy-protected vicinal diol group bound to G by one of the vicinal diol carbon atoms or a disulfide group bound to G by one of the sulfur atoms of the disulfide group;
with the proviso that G has at least 4 carbon atoms when Z represents said disulfide group.

Preferably R is β-cyanoethyl, R¹ and R² are both isopropyl, and G is -(CH₂)ₙ- where n is an integer from 4 to 6, inclusive. Examples of other protecting groups represented by R are β-nitroethyl, 2,2,2-trichloroethyl, methyl, 1,1-dimethyl-2,2,2-trichloroethyl, 2,2,2-tribromoethyl, benzyl, o-chlorophenyl, p-nitrophenylethyl, 2-methylsulfonylethyl, and 1,1-dimethyl-2-cyanoethyl. Examples of other groups which R¹ and R² may represent are other alkyl groups such as butyl, hexyl, nonyl, dodecyl, and hexadecyl, cycloalkyl groups such as cyclopropyl, cyclobutyl, cyclohexyl and cyclooctyl, aryl groups such as phenyl, tolyl, benzyl, xylyl and naphthyl, and when joined together heterocyclic groups such as morpholino, piperidinyl and thiomorpholino. Examples of other hydrocarbylene radicals which G may represent are branched alkylene, and groups containing cycloalkylene (e.g., cyclohexylene) or phenylene. It will be appreciated that G functions primarily as an inert spacer moiety and that it may have substituents and/or heteroatoms (e.g., O, S, N) in its structure that do not affect its ability to act as an inert spacer.

Preferred hydroxy-protected vicinal diol groups represented by Z are those of the formula: 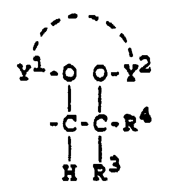 where R³ and R⁴ are individually hydrogen, alkyl of 1 to 20 carbon atoms or monocyclic arylene of 6 to 20 carbon atoms and Y¹ and Y² are individual hydroxy-protecting groups or may be joined (designated by the dashed line) by a single-atom (C, S or Si) bridge to form a five-membered ring protecting group. Y¹ and Y² are of a nature that they are stable during the addition of the molecule to the 5' end of an oligonucleotide chain during chemical synthesis (i.e., conventional automated phosphoramidite synthesis) and can be removed thereafter without damaging the oligonucleotide chain. Further, as discussed below, the vicinal diol structure of the deprotected group permits it to be "activated" by oxidation to convert it from a diol to a functional aldehyde group. Y¹ and Y² may be the same or different and may be any of the individual hydroxy protecting groups that are compatible with conventional automated solid state oligonucleotide chemistry using phosphoramidite chemistry. Examples of such blocking groups are dimethoxytrityl (DMT), trityl, pixyl, benzoyl, acetyl, isobutynyl, p-bromobenzoyl, t-butyldimethylsilyl, and pivaloyl. The protecting groups may be removed with the same or different treatments. Such vicinal diol groups in which R³ and R⁴ are hydrogen and Y¹ and Y² are benzoyl or DMT are particularly preferred.

As indicated, Y¹ and Y² may be linked by a one-atom bridge, thus forming a five-membered ring. Suitable bridging atoms include silicon, sulfur and carbon. It is preferred that the one-atom bridge be a carbon bridge. Thus, the diol group is preferred to be protected as an acetal or ketal, i.e.,

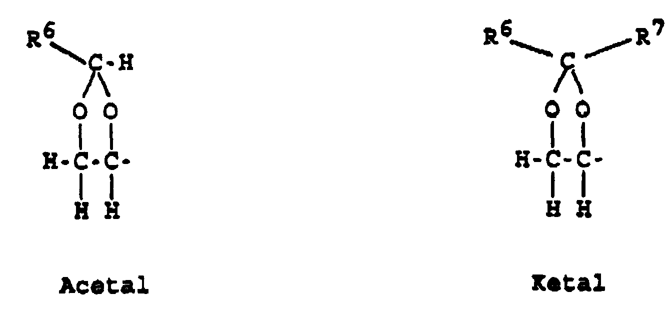

It is important that the bridging atom and its substituents be stable to the subsequent reactions in the sequence used to add the linker to the oligonucleotide. The diol protecting group must also be capable of being removed under mild conditions that do not substantially degrade the oligonucleotide. For example, very acidic conditions will lead to depurination of the oligonucleotide. Suitable groups R⁶ and R⁷ include aryl and substituted aryl groups of 6-30 carbon atoms, C₁-C₂₀ alkyl groups, and aromatic substituted alkyl groups of less than 30 carbon atoms. Preferred is phenyl and phenyl substituted with C₁-C₈ alkyl, C₁-C₈ alkoxy; 1 to 4 atoms of fluorine, chlorine, bromine, nitro- or phenyl. Most preferred are acetal structures wherein R⁶ is phenyl, p-butylphenyl, p-methoxyphenyl, p-tert-butylphenyl, and biphenyl. It will be known to those skilled in the art that the stability of the protecting group can be adjusted for a particular use by a suitable choice of substituent(s).

The above-described acetals and ketals are easily prepared directly from the corresponding triols in one step. It is an important and unexpected feature of this embodiment of the present invention that the vicinal diol is selectively protected in the presence of another free alcohol in the molecule. Thus, the triol wherein Y¹ and Y² are H is simply contacted with an aldehyde to yield the acetal or a ketone to yield the ketal in the presence of an acid catalyst. It is preferred that the contacting take place under conditions where the water formed during the reaction is removed during the reaction, either by the application of vacuum or by solvent azeotrope. Alternatively, acetals or ketals of lower-boiling alcohols can be similarly employed in place of the aldehyde or ketone in an acetal exchange reaction.

The phosphoramidites of the above-described acetals and ketals are prepared by the conventional methods described herein, and they are coupled to the oligonucleotide during the synthesis, as is also described herein. Following the synthesis and purification of the free, coupled oligonucleotide, mild acid hydrolysis of the protecting group generates the diol that is the substrate for the oxidation reaction that produced the aldehyde used for the conjugation reaction. Typical mild hydrolysis conditions are 80% acetic acid/water at 25°C for 30 minutes, similar to those used to remove a dimethoxytrityl group in conventional oligonucleotide synthesis.

Preferred disulfide groups represented by have the formula:

-S-S-R⁵-O-Y³

where R⁵ is an alkylene group of 1 to 20 carbon atoms or a monocyclic arylene groups of 6 to 20 carbon atoms and Y³ is a hydroxy protecting group (as described above).

Most preferably R⁵ is alkylene of 4 to 6 carbon atoms, -OY³ is bound to the ω carbon atom of the alkylene group and Y³ is trityl. As discussed below, the disulfide structure of the group permits it to be "activated" by reduction to cleave the disulfide bond and produce a free sulfhydryl group.

The phosphoramidites wherein Z represents a vicinal diol may be prepared from an alcohol of the formula HC=CH-G-OH. The hydroxyl group of the alcohol is protected and the double bond is oxidized to form the diol group. The hydroxyls of the diol are then protected with an orthogonally removable protecting group (Y² and Y³), i.e., the protecting group on the original hydroxy can be removed without removing the protecting groups on the vicinal diol. The protecting group on the original hydroxy is then removed and the resulting deprotected hydroxy is reacted with an appropriate phosphitylating agent.

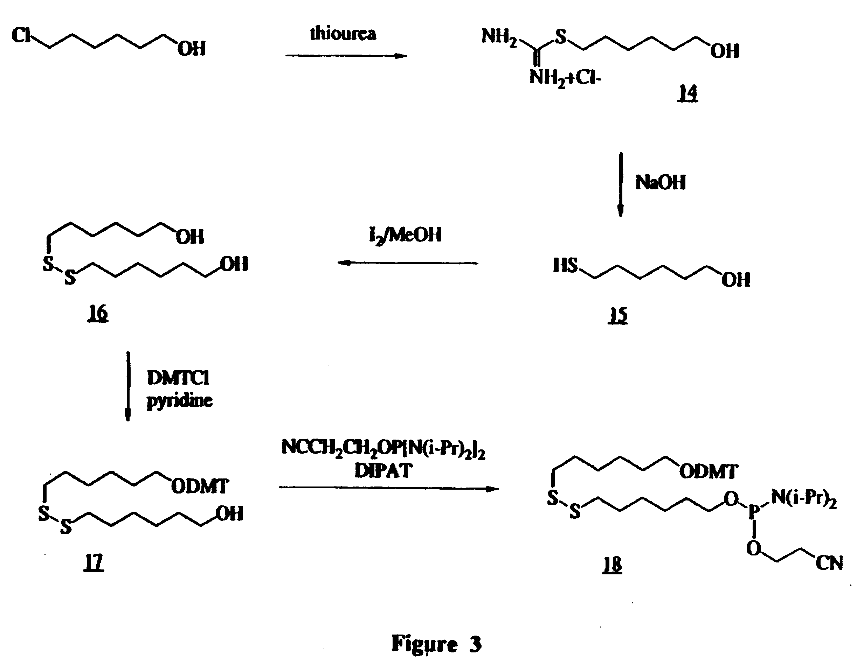

The phosphoramidites wherein Z represents a disulfide may be prepared from symmetrical or asymmetrical disulfides. The general reaction scheme employing symmetrical disulfides is shown in Figure 3 and exemplified by Example 3, infra. Asymmetrical disulfides may be prepared as described by Mannervik, B., and Larson, K., Meth. in Enzym. (1981) 77:420-424, or Mukuiyama, T., and Takahashi, K., Tet Lett (1968) 5907-5908. By way of example, a symmetrical disulfide (HO-G-SS-G-OH) is oxidized with hydrogen peroxide and formic acid to provide the corresponding thiolsulfinate. Treatment of the thiolsulfinate with a mercaptan (e.g., HS-G'-OY³ where Y³ is as described above and G' is a different G than in the starting symmetrical disulfide) at a pH greater than 3 yields an asymmetrical disulfide (HO-G-SS-G'-OY³). This disulfide may be reacted with a phosphitylating agent to yield the phosphoramidate.

The phosphoramidites of the invention may be added to the 5' end of an oligonucleotide chain using the conventional automated phosphoramidite method used to prepare oligonucleotides. See Matteucci, M.D., and Caruthers, M.H., Tet Lett (1980) 521:719, and U.S. Patent No. 4,500,707. The oligonucleotide chain itself may be made by the same method. The length and sequence of the oligonucleotide to which the phosphoramidite of the invention is added will depend upon the use of the resulting 5'-functionalized oligonucleotide. For instance, if the oligonucleotide is to be used for the purposes described in EPA Publication No. 0438259 (i.e., systemic lupus erythematosus (SLE) treatment), then the oligonucleotide will have the ability of bind SLE antibodies. If the oligonucleotide is to be used as a labeled probe then the length and sequence will be such as to be capable of hybridizing to a nucleotide sequence of interest.

As indicated above, the resulting modified oligonucleotide may be represented by the formula: 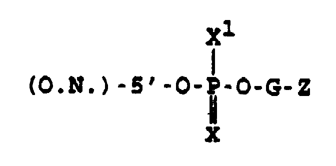 where (O.N.) represents an oligonucleotide chain and X, X¹, G and Z and an defined previously. The designation "5'" indicates that the modifying group is attached to the 5' end of the oligonucleotide chain. The chain will typically be 10 to 200 nucleotides in length, more usually 20 to 60 nucleotides in length.

Once the phosphoramidite has been added to the 5' end of an oligonucleotide chain, the protecting groups (Y¹, Y², Y³) may be removed by appropriate treatment (e.g., base or acid treatment) to yield free hydroxy groups. In the case of the vicinal diol, the diol group is oxidized, e.g., with periodate, to form a terminal aldehyde group. In the case of the disulfide group, the disulfide is reduced with on appropriate reducing agent, e.g., a mercaptan such as dithiothreitol or 2-mercaptoethanol or borohydride to cleave the disulfide bond to form a terminal sulfhydryl group.

The resulting 5' modified oligonucleotide may be coupled via the aldehyde group to labels, carriers, or other molecules having a free amino group or via the sulfhydryl group to an electrophilic center such as maleimide or α-haloacetyl groups or other appropriate Michael acceptors such as acrylates or acrylamides. Examples of such carriers are amino acid polymers such as copolymers of D-lysine and D-glutamic acid, or immunoglobulin, or other polymers that inherently have been derivatized to include such groups as recited above.

### EXAMPLES

The following examples further illustrate the invention. These examples are not intended to limit the invention in any manner. In the examples, Et = ethyl, Ac = acetyl, and THF = tetrahydrofuran.

### EXAMPLE 1

### Preparation of O-(5,6-(bis-O-benzoyloxy)-hexyl)-O-(2-cyanoethyl)-N,N-diisopropylphosphoramidite

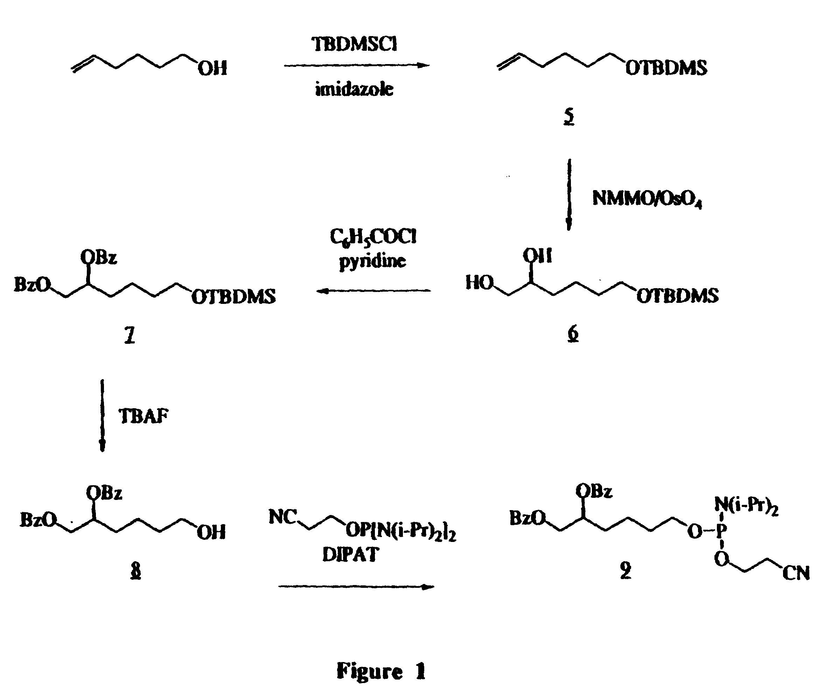

Figure 1 schematically depicts the invention scheme used to make this phosphoramidite. The details of this scheme are described below.

O-(tert-butyldimethylsilyl)-5-hexanol, 5. To a solution of 12.47 mL (10.4 g, 104 mmol) of 5-hexene-1-ol in 104 mL of DMF was added 15.66 g (230 mmol) of imidazole and 20.0 g (130 mmol) of tert-butyldimethylsilyl chloride (TBDMSCl). The mixture was stirred at ambient temperature for 4 hours and partitioned between 200 mL of EtOAc and 100 mL of saturated NaHCO₃ solution. The EtOAc layer was washed with 100 mL of saturated NaHCO₃ solution, 100 mL of saturated NaCl solution, dried (MgSO₄), filtered, and concentrated to a volume of approximately 100 mL. Distillation under vacuum provided 70.07 g of 5: bp 130-143°C @ 100 mmHg; ¹H NMR (CDCl₃) 0.11 (s, 6H), 0.95 (s, 9H), 1.48 (m, 2H), 1.57 (m, 2H), 2.11 (dt, 2H), 3.66 (t, 2H), 5.03 (m, 2H), 5.86 (m, 1H); ¹³C NMR (CDCl₃) -5.25, 18.40, 25.21, 26.01, 32.35, 33.60, 63.09, 114.40, 138.92.

1-O-(tert-butyldimethylsilyl)-1,5,6-hexanetriol, 6. To a solution of 9.86 g (46.0 mmol) of 5 in 92 mL of acetone was added a solution of 6.46 g (55.2 mmol) of N-methylmorpholine oxide (NMMO) in 23 mL of H₂O. To the mixture was added 443 uL of a 2.5% solution of OsO₄ in tert-butyl alcohol (360 mg of solution, 9.0 mg of OsO₄, 35 µmol) and 50 uL of 30% H₂O₂. The mixture was stirred for 16 hours and a solution of 474 mg of sodium dithionite in 14 mL of H₂O was added. After another 0.5 hour the mixture was filtered through celite. The filtrate was dried with MgSO₄ and filtered through 1" of silica gel in a 150 mL Buchner funnel using 250 mL portions of EtOAc to elute. Fractions containing product were concentrated to provide 11.0 g of 6 as a viscous oil: TLC R_{f} 0.2 (1:1 hexane/EtOAc); ¹H NMR (CDCl₃) 0.05 (s, 6H), 0.89 (s, 9H), 1.25 (m, 4H), 1.55 (m, 2H), 3.41 (dd, 2H), 3.62 (t, 2H), 3.71 (m, 1H); ¹³C NMR (CDCl₃) 5.23, 18.42, 21.91, 26.02, 32.68, 32.81, 63.16, 66.74, 72.24.

5,6-(bis-O-benzoyl)-1-O-(tert-butyldimethylsilyl)-1,5,6-hexanetriol, 7. To a solution of 5.29 g (21.3 mmol) of 6 in 106 mL of pyridine was added 6.18 mL (7.48 g, 53.2 mmol) of benzoyl chloride. The mixture was stirred for 18 hours and concentrated on the rotary evaporator. The mixture was partitioned between 100 mL of cold 1 N HCl and 100 mL of EtOAc. This pH of the aqueous layer was checked to made sure it was acidic. The EtOAc layer was washed successively with 100 mL of H₂O and 100 mL of saturated NaCl, dried (MgSO₄), filtered, and concentrated to provide 10.33 g of 7 as a viscous yellow oil; TLC R_{f} 0.45 (1:4 EtOAc/hexanes); ¹H NMR (CDCl₃) 0.05 (s, 6H), 0.88 (s, 9H), 1.59 (m,4H), 1.85 (m, 2H), 3.14 (t, 2H), 4.49 (dd, 1H) 4.59 (dd, 1H), 5.54 (m, 1H), 7.45 (m, 4H), 7.58 (m, 2H), 8.05 (m, 4H).

5,6-(bis-O-benzoyl)-1,5,6-hexanetriol, 8. To a solution of 2.62 g (5.36 mmol) of 7 in 10.9 mL of THF was added 10.7 mL (10.7 mmol) of a 1 N solution of tetrabutylammonium fluoride (CTBAF) in THF. The mixture was allowed to stir for 16 hours. The mixture was partitioned between 25 mL of saturated NaHCO₃ solution and 3 x 25 mL of EtOAc. The combined EtOAc extracts were washed with saturated NaCl solution, dried (MgSO₄), filtered and concentrated to a viscous oil which was purified by silica gel chromatography (1:1 hexane/EtOAc) to provide 823 mg of 8 as a viscous oil; R_{f} .14 (1:1 hexane/ EtOAc); ¹H NMR (CDCl₃) 1.58 (m, 2H), 1.68 (m, 2H), 1.88 (m, 2H), 3.68 (t, 2H), 4.52 (dd, 1H), 4.62 (dd, 1H), 5.56 (m, 1H), 7.46 (m, 4H), 7.58 (m, 2H), 8.05 (m, 4H); ¹³C NMR (CDCl₃) 22.08, 31.20, 31.30, 32.88, 62.92, 66.17, 72.63, 128.93, 130.19, 130.57, 133.62, 166.72, 166.86.

O-(5-6-(bis-O-benzoyloxy)-hexyl)-O-(2-cyanoethyl)-N,N-diisopropylphosphoramidite, 9. To a solution of 1.02 g (2.98 mmol) of 8 and 255 mg (1.49 mg) of diisopropylammonium tetrazolide (DIPAT, prepared by mixing acetonitrile solutions of diisopropylamine and tetrazole in a one-to-one mole ratio and concentrating to a while solid) in 14.9 mL of CH₂Cl₂ was added a solution of 989 mg (3.28 mmol) of O-cyanoethyl-N,N,N',N'-tetraisopropylphosphorodiamidite in 2.0 mL of CH₂Cl₂. The mixture was stirred for 4 hours and partitioned between 25 mL of CH₂Cl₂ and 25 mL of chilled saturated NaHCO₃ solution. The CH₂Cl₂ layer was washed with saturated NaCl solution, dried (Na₂SO₄), filtered, and concentrated. Purification by filtration through a 2" plug of basic alumina in a 25 mm column, eluting with 9:1 EtOAc/Et₃N provided 1.5 g (93%) of 9 as a viscous oil: ¹H NMR (CDCl₃) 1.19 (m, 12H), 1.62 (m, 2H), 1.73 (m, 2H), 1.90 (m, 2H), 2.62 (dd, 2H), 3.53-3.92 (m, 6H), 4.53 (dd, 1H), 4.62 (dd, 1H), 5.58 (m, 1H), 7.48 (m, 4H), 7.60 (m, 2H), 8.09 (m, 4H); ³¹P NMR (CDCl₃ with 15% H₃PO₄ internal standard) 148.2; HRMS (FAB, MH⁺), calculated for C₂₉H₄₀O₆N₂P₁ 543.2624, found 543.2619.

### EXAMPLE 2

### Preparation of O-5-benzyloxy-6-O-(4',4"-dimethoxytrityl) hexyl-O-(2-cyanoethyl)-N,N-diisopropylphosphoramidite

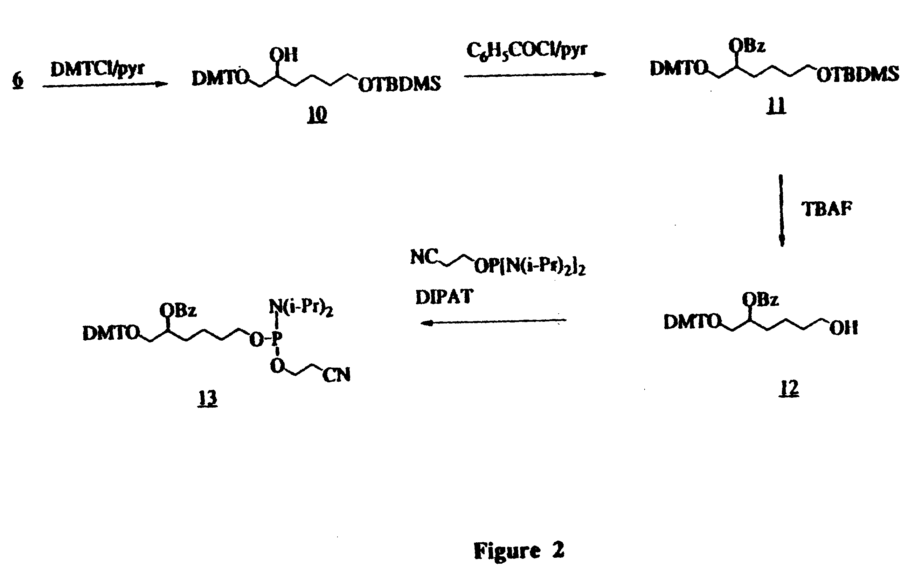

Figure 2 schematically depicts the reaction scheme for making this phosphoramidite. The details of the scheme are described below.

6-O-(4',4"-dimethoxytriphenylmethyl)-1-O-(tert-butyldimethylsilyl)-1,5,6-hexanetriol, 10. To a solution of 1.11 g (4.47 mmol) of 6 and 891 uL (638 mg, 6.30 mmol) of Et₃N in 22 mL of pyridine was added 1.81 g (5.33 mmol) of 4,4'-dimethoxytriphenylmethyl chloride. The mixture was stirred at ambient temperature for 16 hours, concentrated, and purified by silica gel chromatography (29;70:1 EtOAc/hexane/Et₃N) to provide 2.06 g (85%) of 10 as a viscous oil; TLC R_{f} .35 (39:60:1 EtOAc/hexane/Et₃N).

5-O-benzoyl-6-O-(4',4"-dimethoxytriphenylmethyl)-1-O-(tert-butyldimethylsilyl)-1,5,6-hexanetriol. 11. To a solution of 2.06 g (3.8 mmol) of 10 in 19 mL of pyridine was added 532 mL (644 mg, 4.58 mmol) of benzoyl chloride, and the mixture was stirred for 20 hours and concentrated on the rotary evaporator to remove most of the pyridine keeping the bath temperature below 30°C. The mixture was partitioned between 50 mL of EtOAc and 50 mL of saturated NaHCO₃ solution. The EtOAc layer was washed with 50 mL of saturated NaHCO₃ solution, 25 mL of saturated NaCl solution, dried (Na₂SO₄), filtered, and concentrated. Purification by silica gel chrommtoyraphy (10:89:1 EtOAc/hexane/Et₃N) provided 1.66 g of 11 as a viscous oil: TLC R_{f}.27 (1:9 EtOAc/hexane); ¹H NMR (CDCl₃) 0.5 (s, 6H), 0.87 (s, 9H), 1.40 (m, 2H), 1.56 (m, 2H), 1.82 (m, 2H), 3.28 (dd, 2H), 3.60 (t, 2H), 3.80 (s, 6H), 5.38 (m, 1H), 6.79 (m, 4H), 7.17-7.65 (m, 12H), 8.11 (d, 2H).

5-O-benzoyl-6-O-(4',4"-dimethoxytriphenylmethyl)-1,5,6-hexanetriol, 12. To a solution of 1.66 g (2.56 mmol) of 11 in 5.2 mL of THF under N₂ atmosphere was added 5.12 mL (5.12 mmol) of a 1 M solution of tetrabutylammonium fluoride in THF. The mixture was stirred for 3 hours at ambient temperature and concentrated on the rotary evaporator. Purification by silica gel chromatography (1:1 EtOAc/hexane) provided 1.18 g (86%) of 12 as a viscous oil. Further purification was possible by preparative HPLC (12 mL/min, 9:1 MeOH/H₂O, 22.4 mm C₁₈): TLC R_{f}.14 (1:1 hexane/ EtOAc); ¹H NMR (CDCl₃) 1.37 (m, 2H), 1.57 (m, 2H), 1.79 (m, 2H), 3.29 (dd, 2H), 3.60 (t, 2H), 3.75 (s, 6H), 5.36 (m, 1H), 6.80 (m, 4H), 7.17-7.60 (m, 12H), 8.12 (d, 2H).

O-5-benzoyloxy-6-O-(4',4"-dimethoxytriphenylmethyl)hexyl-O-(2'-cyanoethyl)-N,N-diisopropylphosphoramidite,13. To a solution of 681 mg (1.26 mmol) of 12 and 111 mg (0.65 mmol) of diisopropylammonium tetrazolide in 6.5 mL of CH₂Cl₂ was added a solution of 417 mg (1.38 mmol) of O-cyanoethyl-N,N,N',N'-tetraisopropylphosphorodiamidite in 1.0 mL of CH₂Cl₂. The mixture was stirred for 2 hours and partitioned between 25 mL of CH₂Cl₂ and 25 mL of chilled saturated NaHCO₃ solution. The CH₂Cl₂ layer was washed with saturated NaCl solution, dried (Na₂SO₄), filtered, and concentrated. Purification by filtration through a 2" plug of basic alumina in a 25 mm column, eluting with 9:1 CH₂Cl₂/Et₃N provided 798 mg of 13 as a viscous oil: ¹H NMR (CDCl₃) 1.19 (m, 12H), 1.42 (m, 2H), 1.65 (m, 2H), 1.81 (m, 2H), 2.69 (m, 2H), 3.28 (dd, 2H), 3.57 (m, 4H), 3.78 (s, 6H) (underlying m, 2H), 5.40 (m, 1H), 6.79 (dd, 4H), 7.27-7.64 (m, 12H), 8.17 (d, 2H); ³¹P NMR (CDCl₃, 15% H₃PO₄ internal standard) 148.0; HRMS (FAB, MH⁺), calc'd for C₄₃H₅₄O₇N₂P₁ 741.3669, found 741.3678.

### EXAMPLE 3

### Preparation of O-(14-(4',4"-dimethoxytriphenylmethoxy)-7,8-dithiotetradecyl)-O-(2-cyanoethyl)-N-N-diisopropylphosphoramidite

Figure 3 schematically shows the reaction scheme for this phosphoramidite. The details of the scheme are described below.

S-(6-hydroxyhexyl)isothiuronium chloride, 14. To a solution of 16.6 mL (20.0 g, 146 mmol) of 6-chlorohexanol in 49 mL of ethanol was added 11.1 g (146 mmol) of thiourea, and the mixture was refluxed for 24 hours. The mixture was cooled to 0°C, and the product crystallized. The crystals were collected by vacuum filtration and dried to give 28.4 g (92%) of 14 as a white solid: mp 122-124°C; ¹H NMR (DMSO) 1.40 (m, 4H), 1.65 (m, 2H), 3.21 (t, 2H) 3.41 (t, 2H), 9.27 and 9.33 (overlapping broad singlets, 4H).

6-Mercaptohexan-1-ol, 15. To a solution of 17.8 mg (83.6 mmol) of 14 in 120 mL of H₂O and 120 mL of EtOH was added 9.25 g of NaOH pellets. The mixture was refluxed for 4 hours. The mixture was carefully concentrated to approximately 75 mL, and the concentrate was purified by vacuum distillation to provide 7.4 g (66%) of 15: bp 95-105°C @ 5 mm Hg; ¹H NMR (CDCl₃) 1.41 (m, 9H) 2.59 (dt, 2H), 3.69 (t with underlying brd s, 3H).

Bis-(6-hydroxyhexyl)disulfide, 16. To a solution of 4.26 g (31.7 mmol) of 15 in 10 mL of MeOH and 13.7 mL (9.97 g, 98.5 mmol) of Et₃N under N₂ atmosphere and cooled in an ice bath was added dropwise over 10 min a solution of 4.02 g (15.8 mmol) of I₂ in 90 mL of MeOH. The cooling bath was removed, and the mixture was Stirred at ambient temperature for 4 hours. The mixture was concentrated on the rotary evaporator and purified by silica gel chromatography (1:1 hexane/EtOAc) to provide 3.12 g (73%) of 16 as a pale yellow solid: TLC R_{f} .18 (1:1 hexane/EtOAc); mp 38-48°C; ¹H NMR (CDCl₃) 1.15-2.20 (m, 16H), 2.73 (t, 4H), 3.70 (t, 4H).

Mono-O-(4',4"-dimethoxytriphenylmethyl)-bis-(6-hydroxyhexyl)disulfide, 17. To a solution of 3.12 g (11.7 mmol) of 16 and 45 mL of pyridine was added 3.97 g (11.7 mmol of 4,4'-dimethoxytriphenylmethyl chloride, and the mixture was stirred at ambient temperature for 16 hours. Most of the pyridine was removed on the rotary evaporator, and the residue was partitioned between 100 mL of saturated NaHCO₃ solution and 100 mL of EtOAc. The EtOAc layer was washed with 50 mL of saturated NaCl solution, dried (Na₂SO₄), filtered and concentrated to an oil. Purification by silica gel chromatography (9:1 CH₂Cl₂/EtOAc) yielded 2.84 g (43%) of 17 as a viscous oil: TLC R_{f} .35 (9:1 CH₂Cl₂/EtOAc); ¹H NMR (CDCl₃) 1.41 (m, 8H), 1.65 (m, 8H), 2.70 (two overlapping triplets, 4H), 3.08 (t, 2H), 3.65 (t, 2H), 3.81 (s, 6H), 6.85 (d, 4H), 7.32 (m, 7H), 7.47 (d, 2H).

O-(14-(4',4"-Dimethoxytriphenylmethoxy)-7,8-dithiotetradecyl)-O-(2-cyanoethyl)-N,N'-diisopropylphosphoramidite, 18. To a solution of 771 mg (1.36 mmol) of 17 and 116 mg (0.68 mmol) of diisopropylammonium tetrazolide in 6.8 mL of CH₂Cl₂ under N₂ atmosphere was added a solution of 458 mg (1.52 mmol) of O-cyanoethyl-N,N,N',N'-tetraisopropylphosphorodiamidite in 0.5 mL of CH₂Cl₂.The mixture was stirred for 4 h and partitioned between 25 mL of NaHCO₃ and 3 x 25 mL of CH₂Cl₂. The combined CH₂Cl₂ layers were washed with saturated NaCl solution, dried (Na₂CO₃), filtered and concentrated to an oil. Purification by filtration through a 2" plug of basic alumina in a 25 mm column, eluting with 9:1 CH₂Cl₂/Et₃N provided 831 mg (80%) of 18 as a viscous oil; ¹H NMR (CDCl₃) 1.25 (m, 12H), 1.45 (m, 8H), 1.70 (m, 8H), 2.72 (m, 6H), 3.09 (t, 2H), 3.65 (m, 4H), 3.87 (s, 6H) 3.91 (m, 2H), 6.89 (d, 4H), 7.35 (m, 7H), 7.49 (d, 2H); ³¹P NMR (CDCl₃ with 15% H₃PO₄ internal standard) 147.69; HRMS (FAB, MH⁺) calc'd for C₄₂H₆₂N₂O₅P₁S₂ 769.3839, found 769.3853.

### EXAMPLE 4

### Addition of Phosphoramidite of Example 1 to Oligonucleotide

A fivefold molar excess (760 mg) of the phosphoramidite of Example 1 was coupled to the 5' end of an oligonucleotide which was attached to 10 g (300 µmoles) CPG (control pore glass) support. This synthesis was performed on a Milligen 8800 DNA synthesizer using the manufacturer's protocols for DNA synthesis.

In a separate instance, in a 1 µmole scale reaction on a Pharmacia Gene-Assembler DNA synthesizer, the coupling efficiency was determined to 96% by trityl release. For this determination, the phosphoramidite from Example 3 was used.

After the reaction, the CPG was suspended in 100 ml concentrated ammonia and kept at 55°C overnight. After filtration, the deprotected oligonucleotide was purified by sodium chloride gradient and ion-exchange chromatography.

The fractions were analyzed by polyacrylamide gel electrophoresis and the product containing fractions pooled, adjusted to 0.3 M NaCl with 3 M NaCl solution and precipitated by the addition of an equal volume of cold isopropanol. The product was collected by centrifugation and dried in vacuo.

The pellet was then dissolved in 40 ml water and oxidized by treatment with a fivefold molar excess of sodium metaperiodate (83.6 mg for 2 g purified oligonucleotide in this example) at 0°C for 30 min. The solution was again adjusted to 0.3 M NaCl and precipitated as above to remove the formaldehyde produced in this reaction. After centrifugation and drying, this material wa used in the next step.

### EXAMPLE 5

### Conjugation of Oligonucleotide of Example 4 to D-glutamic acid, D-lysine (DEK) Polymer

100 mg of oxidized oligonucleotide (2.5 µmoles) was dissolved in 1.33 ml of 100 mM NaBO₃, pH 8.0. Then, 2.5 mg of DEK (0.25 µmoles, MWt 10,000, 60:40 weight ratio of D-glutamic acid to D-lysine) and 0.79 mg NaCNBH₃ (12.5 µmoles) was added. The mixture (2.0 ml) was incubated at 37°C for 3 days. The condensation product was purified by S-200 (Pharmacia, Uppsala, Sweden) chromatography.

The fractions were labeled with alpha ³²P ddATP and terminal transferase for viewing on a standard 8% DNA sequencing polyacrylamide gel.

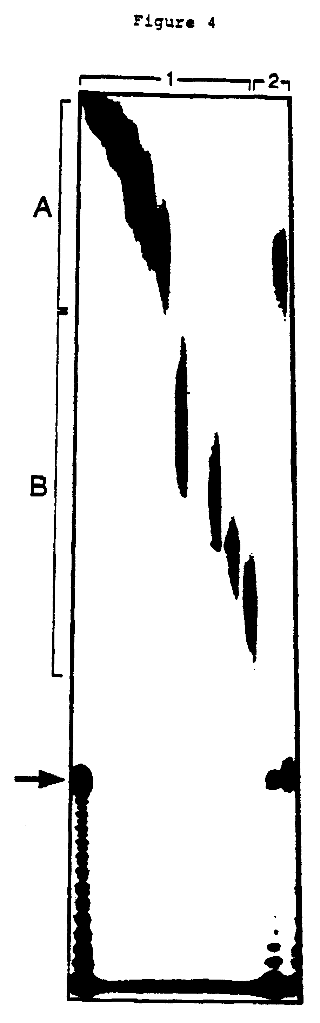

The various radiolabeled fractions were visualized by electrophoresis and autoradiography as presented in Figure 4. The lanes labeled "2" contain unconjugated full length oligonucleotide and the arrow indicates the position of the 50-mer. Lanes labeled "1" contain conjugates of decreasing molecular weight. Fractions which contain the higher substitute (region A) oligo-DEK conjugate were pooled for subsequent annealing to the complementary oligonucleotide strand to construct a double stranded DNA-DEK conjugate.

### EXAMPLE 6

### Conjugation of Oligonucleotide of Example 4 to Keyhole Limpet Hemocyanin (KLH)

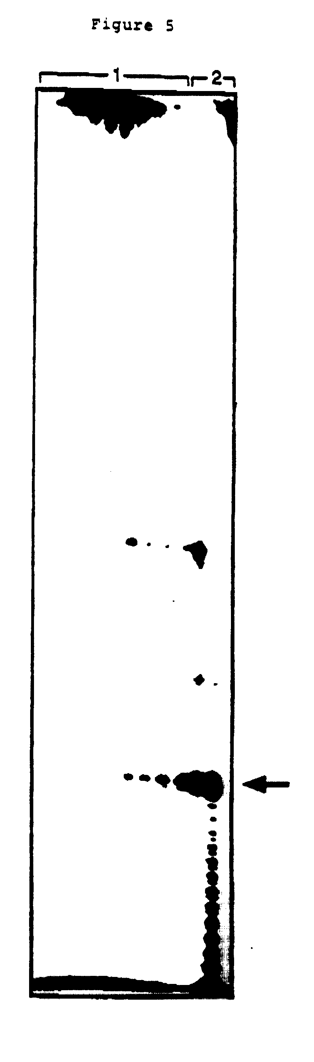

100 mg crude oxidized oligonucleotide (2.5 µmoles) was dissolved in 1.33 ml of 50 mM NaBO₃, pH 8.0. Then, 31.3 mg of KLH (0.208 µmoles) and 2.0 mg NaCNBH₃ (31.8 µmoles) was added. The mixture (2.0 ml) was incubated at 37°C for 3 days. The condensation product was purified by S-200 chromatography. The various fractions were radiolabeled using the same process as described above for D-EK and were then visualized after electrophoresis and autoradiography as presented in Figure 5. Lanes labeled "1" are high molecular weight conjugates, lanes labeled "2" contain mostly unconjugated oligo and the arrow indicates the position of the 50-mer. Modifications of the above-describes modes for carrying out the invention that are obvious to those of ordinary skill in the fields of organic chemistry, and particularly oligonucleotide synthesis and derivatization are intended to be within the scope of the following claims. The fractions which contained the oligo-KLH conjugate were pooled for subsequent annealing to the complimentary oligonucleotide strand to construct a double-stranded DNA-KLH conjugate.

### EXAMPLE 7

### Preparation of Acetal-Protected Diol Phosphoramidite 4-(4-hydroxy-1-butyl)-2-phenyl-1,3 dioxolane.

A mixture of 1,2,6-trihydroxyhexane (2.58 g) and benzaldehyde dimethyl acetal (3.18 g) is treated with toluene sulfonic acid hydrate (2.08 g). The mixture is allowed to stir at room temperature for 60 hours, and is then partitioned between saturated aqueous sodium bicarbonate (50 ml) and methylene chloride (20 ml). The layers are separated, the aqueous layer is re-extracted with methylene chloride, the organic layers are dried over anhydrous sodium sulfate, filtered, and concentrated to an oil (2.66 g), which is purified by column chromatography (silica gel, 1:1 ethyl acetate/hexanes). Pooling and concentrating the appropriate fractions give the title compound as an oil (1.19 g): TLC Rf = 0.18 (silica, 1:1 ethyl acetate/hexanes); ¹H NMR (CDCl₃), δ, 1.62 (m, 6H), 3.67 (m, 3H), 3.25 (m, 2H), 6.37 (s, 0.6H), 6.50 (s, 0.4H), 8.04 (br, s, 5H).

In a similar manner, but beginning with benzaldehyde in place of benzaldehyde dimethyl acetal, the title compound is also obtained.

(4-(2-phenyl-1,3-dioxol-4-yl) butyl)-O-(2-cyanoethyl)-N,N-diisopropylphosphoramidite. A solution of the above dioxolane (1.19 g), and diisopropylamine (2.0 ml) in methylene chloride (22 ml) is treated with cyanoethyldiisopropylchlorophosphoramidite (0.92 ml) and allowed to stir at 24°C for 1.5 hours. The mixture is partitioned between saturated aqueous sodium bicarbonate (25 ml) and methylene chloride (25 ml). The layers are separated, the aqueous layer is re-extracted with methylene chloride, the organic layers are dried over anhydrous sodium sulfate, filtered, and concentrated to an oil (2.13 g), which is purified by column chromatography (basic alumina, 1:1 methylene chloride/hexanes, 1% triethylamine). Pooling and concentrating the appropriate fractions gives the title compound as an oil (1.28 g): ¹H NMR (CDCl₃), δ, 1.13 (12H), 1.5-1.9 (m, 8H), 2.58 (q, 2H), 3.5-3.8 (m, 8H), 4.0-4.3 (m, 2H), 5.8 (s, 0.6H), 5.92 (s, 0.4H), 7.3-7.5 (m, 5H).

In a similar manner, the following phosphoramidites are prepared: (4-(2-methoxyphenyl-1,3-dioxol-4-yl) butyl)-O-(2-cyanoethyl)-N,N-diisopropylphosphoramidite; (4-(2-p-butylphenyl-1,3-dioxol-4-yl)butyl)-O-(2-cyanoethyl)-N,N-diisopropylphosphoramidite;(4-(2-biphenyl-1-3-dioxol-4-yl) butyl)-O-(2-cyanoethyl)-N,N-diisopropylphosphoramidite; (4-(2-methyl-2-phenyl-1,3-dioxol-4-yl) butyl)-O-(2-cyanoethyl)-N,N-diisopropylphosphoramidite.

### EXAMPLE 8

### Addition of Phosphoramidite of Example 7 to Oligonucleotide

In the manner of Example 4, the phosphoramidite of Example 7 is coupled to the oligonucleotide. Following purification, the acetal protecting group is removed with 80% acetic acid/water for 40 minutes. The progress of the reaction is monitored by HPLC using a Gen Pak Fax column (Waters Associates), using 0.5M sodium phosphate at pH 7.5, with a 1.0M sodium chloride/10% methanol gradient. The starting acetal elutes at 20.1 minutes, and the hydrolyzed diol elutes at 18.9 minutes.

Modifications of the above-described modes for carrying out the invention that are obvious to those of skill in the fields of organophosphorous chemistry, nucleotide chemistry, oligonucleotide synthesis, or related fields are intended to be within the scope of the following claims.

## Claims (Claims for the following Contracting State(s): AT, BE, CH, LI, DK, DE, FR, GB, IT, LU, MC, NL, PT, SE)

1. A compound of the formula: 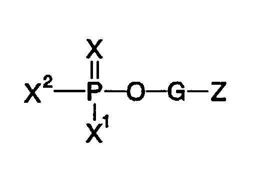 where
X is:
(i) an oxygen or sulphur atom when X¹ represents O⁻, methyl or -OCH₃ and X² represents a hydrogen atom or RO⁻ where R represents a protecting group;
(ii) not present when
(a) X¹ represents a chlorine atom and X² represents methyl or RO-; or
(b) X² represents RO- and X¹ represents NR¹R² where each R¹ and R² individually represents C₁₋₆ alkyl, C₃₋₈ cycloalkyl or a C₆₋₂₀ aryl group or, when joined together, form with the nitrogen atom a C₄₋₇ cyclic structure with 0 or 1 oxygen or sulphur atoms;
G represents a C₁₋₂₀ hydrocarbylene group; and
Z represents a hydroxy protected vicinal diol group bound to G by one of the vicinal diol carbon atoms or a disulfide group bound to G by one of the sulfur atoms of the disulfide group;
with the proviso that G has at least 4 carbon atoms when Z represents said disulfide group.

2. A compound of the formula: 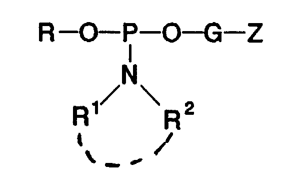
where R represents a methyl group or base-labile protecting group;
each R¹ and R² individually represents a C₁₋₆ alkyl, C₃₋₈ cycloalkyl, or a C₆₋₂₀ aryl group or, when joined together, form with the nitrogen atom C₄₋₇ cyclic structure having 0 or 1 oxygen or sulphur atoms;
G represents a C₁₋₂₀ hydrocarbylene group; and
Z represents a hydroxy-protected vicinal diol group bound to G by one of the vicinal diol carbon atoms or a disulfide group bound to G by one of the sulfur atoms of the disulfide group;
with the proviso that G has at least 4 carbon atoms when Z represents said disulfide group.

3. A compound according to claim 1 or 2 where Z has the formula: 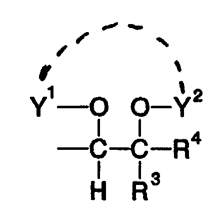
where each R³ and R⁴ individually represents a hydrogen atom, a C₁₋₂₀ alkyl or C₆₋₂₀ monocyclic aryl group, and each Y¹ and Y² individually represents a hydroxy-protecting group or are together joined by a single-atom bridge to form a five-membered ring protecting group;
or Z represents
-S-S-R⁵-O-Y³
where R⁵ represents a C₁₋₂₀ alkylene group or a C₆₋₂₀ monocyclic arylene group and Y³ is a hydroxy protecting group.

4. A compound according to claim 3 wherein R⁵ represents a C₄₋₆ alkylene group, -OY³ is bound to the ω atom of the alkylene group and Y³ is trityl.

5. A compound according to any preceding claim wherein X¹ represents NR¹R² and R¹ and R² are both isopropyl.

6. A compound according to claim 3 or 5 wherein R³ and R⁴ are both hydrogen, R⁵ is -(CH₂)_{6'} and Y¹, Y² and Y³ are all benzoyl or dimethoxytrityl.

7. A compound according to any of the preceding claims wherein X¹ represents NR¹R² and R¹ and R² are both isopropyl, G is -(CH₂)₄, and either R is β-cyanoethyl or Z has the formula: 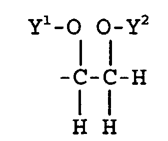
where Y¹ and Y² are both benzoyl or Y¹ is benzoyl and Y² is dimethoxytrityl.

8. A compound according to claim 1 or 2 wherein Z represents a hydroxy protected vicinal diol group of the formula: 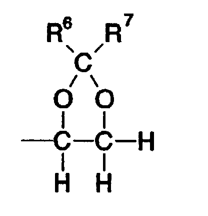
wherein each R⁶ and R⁷ individually represents a hydrogen atom, C₆₋₃₀ aryl or substituted aryl, C₁₋₂₀ alkyl or an aromatic-substituted alkyl group of less than 30 carbon atoms;
with the proviso that R⁶ and R⁷ can not both be hydrogen atoms.

9. A compound according to claim 8 wherein each R⁶ and R⁷ individually represents a hydrogen atom or phenyl optionally substituted with C₁₋₈ alkyl, C₁₋₈ alkoxy, 1 to 4 halogen atoms each of atomic number of from 9 to 35, nitro-, or phenyl, with the proviso that R⁶ and R⁷ can not both be hydrogen atoms.

10. A compound according to claim 8 or 9 wherein R⁶ represents a hydrogen atom and R⁷ represents a phenyl, p-butylphenyl, p-methoxyphenyl, p-t-butylphenyl or biphenyl group.

11. A compound according to claim 1 or 2 wherein X¹ represents NR¹R² and both R¹ and R² are isopropyl, G is -(CH₂)₆-, and Z has the formula:
-S-S-R⁵-O-Y³
where R⁵ is -(CH₂)₆- and Y³ is dimethoxytrityl.

12. A 5' -modified oligonucleotide of the formula: 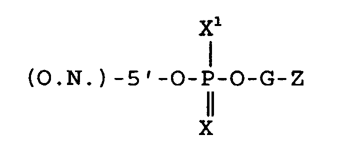
where (O.N.) represents an oligonucleotide chain;
X is an oxygen or sulphur atom;
X¹ represents O⁻, methyl, -OCH₃ or NR¹R² where each R¹ and R² individually represent a hydrogen atom or a C₁₋₆ alkyl, C₃₋₈ cycloalkyl or C₆₋₂₀ aryl group or, when joined together, form with the nitrogen atom a C₄₋₇ cyclic structure containing none or one oxygen or sulphur atoms; G is a C₁₋₂₀ hydrocarbylene group; and
Z is a hydroxy-protected vicinal diol group bound to G by one of the vicinal diol carbon atoms or a disulfide group bound to G by one of the sulfur atoms of the disulfide group.

13. A 5' -modified oligonucleotide according to claim 12 wherein Z has the formula: 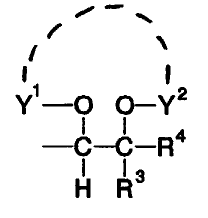
where each R³ and R⁴ individually represents a hydrogen atom, a C₄₋₂₀ alkyl or C₆₋₂₀ monocyclic aryl group and each Y¹ and Y² individually represents a hydroxy protecting group or are together joined by a single-atom bridge to form a five-membered ring protecting group, or
-S-S-R⁵-O-Y³
where R⁵ represents a C₁₋₂₀ alkylene group or a C₆₋₂₀ monocyclic arylene group and Y³ is a hydroxy-protecting group.

14. A 5' -modified oligonucleotide according to claim 13 wherein Y¹ and Y² are removed to leave free hydroxy groups.

15. A 5'-modified oligonucleotide according to claim 13 wherein G is -(CH₂)₄-, and Z has the formula: 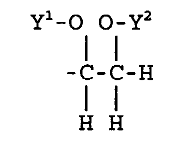
where Y¹ and Y² are both benzoyl or Y¹ is benzoyl and Y² is dimethoxytrityl.

16. A 5'-modified oligonucleotide according to claim 15 wherein Y¹ and Y² are removed to leave free hydroxyl groups.

17. A 5'-modified oligonucleotide according to claim 12 or 13 wherein Z has the formula: 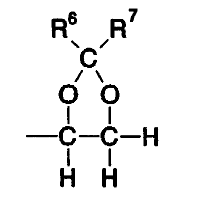
wherein each R⁶ and R⁷ individually represent a hydrogen atom, C₆₋₃₀ aryl or substituted aryl, C₁₋₂₀ alkyl or aromatic-subtituted alkyl of less than 30 carbon atoms;
with the proviso that R⁶ and R⁷ can not both be hydrogen atoms.

18. A 5'-modified oligonucleotide according to claim 17 wherein each R⁶ and R⁷ individually represents a hydrogen atom or phenyl optionally substituted with a C₁₋₈ alkyl, C₁₋₈ alkoxy, 1 to 4 halogen atoms each of atomic number 9 to 35, nitro-, or phenyl, with the proviso that both R⁶ and R⁷ can not both be hydrogen atoms.

19. A 5' -modified oligonucleotide according to claim 17 or 18 wherein R⁶ is a hydrogen atom and R⁷ represents a phenyl, p-butylphenyl, p-methoxyphenyl, p-t-butylphenyl or biphenyl group.

20. A 5' -modified oligonucleotide according to claim 12 or 13 wherein G is -(CH₂)₆-, and Z has the formula:
-S-S-R⁵-O-Y³
where R⁵ is -(CH₂)₆- and Y³ is dimethoxytrityl.

21. A 5' -modified oligonucleotide according to claim 14 or 16 wherein Z has been oxidized to form a terminal aldehyde group on the oligonucleotide.

22. A 5' -modified oligonucleotide according to claim 20 wherein Z has been reduced to form a terminal thiol group on the oligonucleotide.

23. A conjugate of an amino group-containing carrier molecule and a 5' -modified oligonucleotide according to claim 21, wherein the conjugate is formed by reaction between said amino group and said terminal aldehyde group.

24. A conjugate of a carrier molecule having a functional group and a 5' -modified oligonucleotide according to claim 22, wherein the conjugate is formed by reaction between said functional group and said terminal thiol group.

25. A conjugate according to claim 23 or 24 wherein the carrier molecule is a polymer.

26. A conjugate according to claim 25 wherein the polymer is an amino acid polymer.

27. A partially protected alcohol of the formula: 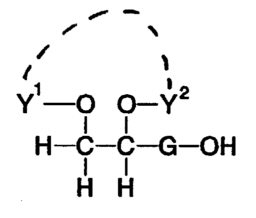
where each Y¹ and Y² individually represents a hydroxy protecting group or are together joined by a single-atom bridge, other than a -C(=O)- bridging group, to form a five-membered ring protecting group and G represents a C₂₋₂₀ hydrocarbylene group.

28. An alcohol according to claim 27 wherein both Y¹ and Y² are benzoyl and G is butylene.

29. An alcohol according to claim 27 wherein Y¹ is dimethoxytrityl, Y² is benzoyl and G is butylene.

30. An alcohol according to claim 27 wherein each Y¹ and Y² individually represents: 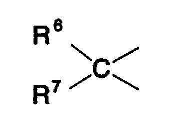
wherein each R⁶ and R⁷ individually represents a hydrogen atom, C₆₋₃₀ aryl or substituted aryl, C₁₋₂₀ alkyl or an aromatic-substituted alkyl group of less than 30 carbon atoms, with the proviso that both R⁶ and R⁷ can not both be hydrogen atoms.

31. A disulfide of the formula
Y³-O-G-S-S-G-OH
wherein G represents a C₄₋₂₀ hydrocarbylene group and Y³ represents a hydroxyl protecting group.

32. A disulfide according to claim 31 wherein Y³ is dimethoxytrityl and G is hexylene.

## Claims (Claims for the following Contracting State(s): ES, GR)

1. A process for modifying an oligonucleotide comprising reacting an oligonucleotide with a compound of the formula: 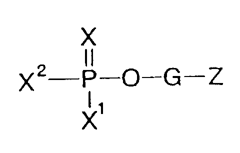 where
X is:
(i) an oxygen or sulphur atom when X¹ represents O⁻, methyl or -OCH₃ and X² represents a hydrogen atom or RO- where R represents a protecting group;
(ii) not present when
(a) X¹ represents a chlorine atom and X² represents methyl or RO-; or
(b) X² represents RO- and X¹ represents NR¹R² where each R¹ and R² individually represents C₁₋₆ alkyl, C₃₋₈ cycloalkyl or a C₆₋₂₀ aryl group or, when joined together, form with the nitrogen atom a C₄₋₇ cyclic structure with 0 or 1 oxygen or sulphur atoms;
G represents a C₁₋₂₀ hydrocarbylene group; and
Z represents a hydroxy protected vicinal diol group bound to G by one of the vicinal diol carbon atoms or a disulfide group bound to G by one of the sulfur atoms of the disulfide group;
with the proviso that G has at least 4 carbon atoms when Z represents said disulfide group;
to produce a 5' modified oligonucleotide.

2. A process for modifying an oligonucleotide comprising reacting an oligonucleotide with a compound of the formula: 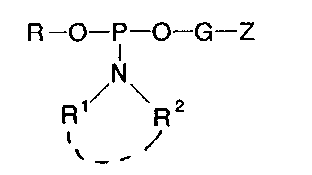
where R represents a methyl group or base-labile protecting group;
each R¹ and R² individually represents a C₁₋₆ alkyl, C₃₋₈ cycloalkyl, or a C₆₋₂₀ aryl group or, when joined together, form with the nitrogen atom C₄₋₇ cyclic structure having 0 or 1 oxygen or sulphur atoms;
G represents a C₁₋₂₀ hydrocarbylene group; and
Z represents a hydroxy-protected vicinal diol group bound to G by one of the vicinal diol carbon atoms or a disulfide group bound to G by one of the sulfur atoms of the disulfide group;
with the proviso that G has at least 4 carbon atoms when Z represents said disulfide group,
to produce a 5' modified oligonucleotide.

3. A process according to claim 1 or 2 where Z has the formula: 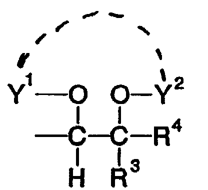
where each R³ and R⁴ individually represents a hydrogen atom, a C₁₋₂₀ alkyl or C₆₋₂₀ monocyclic aryl group, and each Y¹ and Y² individually represents a hydroxy-protecting group or are together joined by a single-atom bridge to form a five-membered ring protecting group;
or Z represents
-S-S-R⁵-O-Y³
where R⁵ represents a C₁₋₂₀ alkylene group or a C₆₋₂₀ monocyclic arylene group and Y³ is a hydroxy protecting group.

4. A process according to claim 3 wherein R⁵ represents a C₄₋₆ alkylene group, -OY³ is bound to the ω atom of the alkylene group and Y³ is trityl.

5. A process according to any preceding claim wherein X¹ represents NR¹R² and R¹ and R² are both isopropyl.

6. A process according to claim 3 or 5 wherein R³ and R⁴ are both hydrogen, R⁵ is -(CH₂)₆ and Y¹, Y² and Y³ are all benzoyl or dimethoxytrityl.

7. A process according to any of the preceding claims wherein X¹ represents NR¹R² and R¹ and R² are both isopropyl, G is -(CH₂)₄, and either R is β-cyanoethyl or Z has the formula: 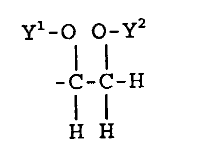
where Y¹ and Y² are both benzoyl or Y¹ is benzoyl and Y² is dimethoxytrityl.

8. A process according to claim 1 or 2 wherein Z represents a hydroxy protected vicinal diol group of the formula: 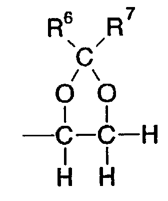
wherein each R⁶ and R⁷ individually represents a hydrogen atom, C₆₋₃₀ aryl or substituted aryl, C₁₋₂₀ alkyl or an aromatic-substituted alkyl group of less than 30 carbon atoms;
with the proviso that R⁶ and R⁷ cannot both be hydrogen atoms.

9. A process according to claim 8 wherein each R⁶ and R⁷ individually represents a hydrogen atom or phenyl optionally substituted with C₁₋₈ alkyl, C₁₋₈ alkoxy, 1 to 4 halogen atoms each of atomic number of from 9 to 35, nitro-, or phenyl, with the proviso that R⁶ and R⁷ can not both be hydrogen atoms.

10. A process according to claim 8 or 9 wherein R⁶ represents a hydrogen atom and R⁷ represents a phenyl, p-butylphenyl, p-methoxyphenyl, p-t-butylphenyl or biphenyl group.

11. A process according to claim 1 or 2 wherein X¹ represents NR¹R² and both R¹ and R² are isopropyl, G is -(CH₂)₆-, and Z has the formula:
-S-S-R⁵-O-Y³
where R⁵ is -(CH₂)₆ and Y³ is dimethoxytrityl.

12. A process according to any preceding claim further comprising the step of removing the hydroxy protecting groups to leave free hydroxy groups.

13. A process according to claim 12 further comprising the step of oxidising Z to form a terminal aldehyde group on the oligonucleotide.

14. A process according to claim 11 further comprising the step of reducing Z to form a terminal thiol group on the oligonucleotide.

15. A process according to claim 13 further comprising the step of conjugating an amino-containing carrier molecule with said terminal aldehyde group.

16. A process according to claim 14 further comprising the step of conjugating a carrier molecule containing a functional group with said terminal thiol group.

17. A process according to claim 15 or 16 wherein the carrier molecule is a polymer.

18. A process according to claim 17 wherein the polymer is an amino acid polymer.

19. A compound of the formula: 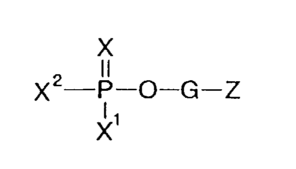 where
X is:
(i) an oxygen or sulphur atom when X¹ represents O⁻, methyl or -OCH₃ and X² represents a hydrogen atom or RO- where R represents a protecting group;
(ii) not present when
(a) X¹ represents a chlorine atom and X² represents methyl or RO-; or
(b) X² represents RO- and X¹ represents NR¹R² where each R¹ and R² individually represents C₁₋₆ alkyl, C₃₋₈ cycloalkyl or a C₆₋₂₀ aryl group or, when joined together, form with the nitrogen atom a C₄₋₇ cyclic structure with 0 or 1 oxygen or sulphur atoms;
G represents a C₁₋₂₀ hydrocarbylene group; and
Z represents a hydroxy protected vicinal diol group bound to G by one of the vicinal diol carbon atoms or a disulfide group bound to G by one of the sulfur groups of the disulfide group;
with the proviso that G has at least 4 carbon atoms when Z represents said disulfide group.

20. A compound of the formula: 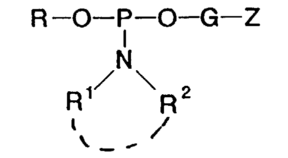
where R represents a methyl group or base-labile protecting group;
each R¹ and R² individually represents a C₁₋₆ alkyl, C₃₋₈ cycloalkyl, or a C₆₋₂₀ aryl group or, when joined together, form with the nitrogen atom C₄₋₇ cyclic structure having 0 or 1 oxygen or sulphur atoms;
G represents a C₁₋₂₀ hydrocarbylene group; and
Z represents a C₁₋₂₀ hydroxy-protected vicinal diol group bound to G by one of the vicinal diol carbon atoms or a disulfide group bound to G by one of the sulfur atoms of the disulfide group;
with the proviso that G has at least 4 carbon atoms when Z represents said disulfide group.

21. A compound according to claim 19 or 20 where Z has the formula: 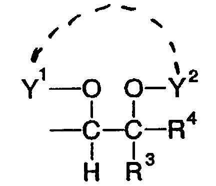
where each R³ and R⁴ individually represents a hydrogen atom, a C₁₋₂₀ alkyl or C₆₋₂₀ monocyclic aryl group, and each Y¹ and Y² individually represents a hydroxy-protecting group or are together joined by a single-atom bridge to form a five-membered ring protecting group;
or Z represents
-S-S-R⁵-O-Y³
where R⁵ represents a C₁₋₂₀ alkylene group or a C₆₋₂₀ monocyclic arylene group and Y³ is a hydroxy protecting group.

22. A compound according to claim 21 wherein R⁵ represents a C₄₋₆ alkylene group, -OY³ is bound to the ω atom of the alkylene group and Y³ is trityl.

23. A compound according to any of claims 19-22 wherein X¹ represents NR¹R² and R¹ and R² are both isopropyl.

24. A compound according to claim 21 or 23 wherein R³ and R⁴ are both hydrogen, R⁵ is -(CH₂)₆, and Y¹, Y² and Y³ are all benzoyl or dimethoxytrityl.

25. A compound according to any of claims 19-24 wherein X¹ represents NR¹R² and R¹ and R² are both isopropyl, G is -(CH₂)₄, and either R is β-cyanoethyl or Z has the formula: 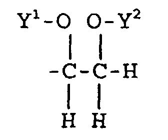
where Y¹ and Y² are both benzoyl or Y¹ is benzoyl and Y² is dimethoxytrityl.

26. A compound according to claim 19 or 20 wherein Z represents a hydroxy protected vicinal diol group of the formula: 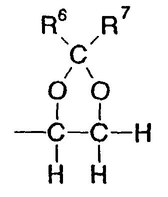
wherein each R⁶ and R⁷ individually represents a hydrogen atom, C₆₋₃₀ aryl or substituted aryl, C₁₋₂₀ alkyl or an aromatic-substituted alkyl group of less than 30 carbon atoms;
with the proviso that R⁶ and R⁷ can not both be hydrogen atoms.

27. A compound according to claim 26 wherein each R⁶ and R⁷ individually represents a hydrogen atom or phenyl optionally substituted with C₁₋₈ alkyl, C₁₋₈ alkoxy, 1 to 4 halogen atoms each of atomic number of from 9 to 35, nitro-, or phenyl, with the proviso that R⁶ and R⁷ can not both be hydrogen atoms.

28. A compound according to claim 27 or 28 wherein R⁶ represents a hydrogen atom and R⁷ represents a phenyl, p-butylphenyl, p-methoxyphenyl, p-t-butylphenyl or biphenyl group.

29. A compound according to claim 19 or 20 wherein X¹ represents NR¹R² and both R¹ and R² are isopropyl, G is -(CH₂)₆-, and Z has the formula:
-S-S-R⁵-O-Y³
where R⁵ is -(CH₂)₆- and Y³ is dimethoxytrityl.

30. A 5' -modified oligonucleotide of the formula: 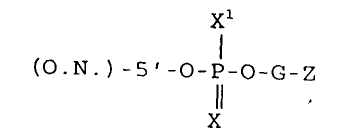 where (O.N.) represents an oligonucleotide chain;
X is an oxygen or sulphur atom;
X¹ represents O⁻, methyl, -OCH₃ or NR¹R² where each R¹ and R² individually represent a hydrogen atom or a C₁₋₆ alkyl, C₃₋₈ cycloalkyl or C₆₋₂₀ aryl group or, when joined together, form with the nitrogen atom a C₄₋₇ cyclic structure containing none or one oxygen or sulphur atoms; G is a C₁₋₂₀ hydrocarbylene group; and
Z is a hydroxy-protected vicinal diol group bound to G by one of the vicinal diol carbon atoms or a disulfide group bound to G by one of the sulfur atoms of the disulfide group.

31. A 5' -modified oligonucleotide according to claim 30 wherein Z has the formula: 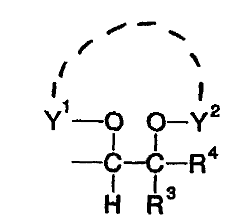
where each R³ and R⁴ individually represents a hydrogen atom, a C₄₋₂₀ alkyl or C₆₋₂₀ monocyclic aryl group and each Y¹ and Y² individually represents a hydroxy protecting group or are together joined by a single-atom bridge to form a five-membered ring protecting group, or
-S-S-R⁵-O-Y³
where R⁵ represents a C₁₋₂₀ alkylene group or a C₆₋₂₀ monocyclic arylene group and Y³ is a hydroxy-protecting group.

32. A 5' -modified oligonucleotide according to claim 31 wherein Y¹ and Y² are removed to leave free hydroxy groups.

33. A 5' -modified oligonucleotide according to claim 31 wherein G is -(CH₂)₄-, and Z has the formula: 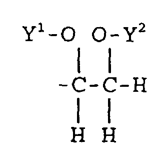
where Y¹ and Y² are both benzoyl or Y¹ is benzoyl and Y² is dimethoxytrityl.

34. A 5' -modified oligonucleotide according to claim 33 wherein Y¹ and Y² are removed to leave free hydroxyl groups

35. A 5' -modified oligonucleotide according to claim 30 or 31 wherein Z has the formula: 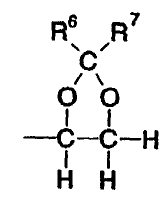
wherein each R⁶ and R⁷ individually represent a hydrogen atom, C₆₋₃₀ aryl or substituted aryl, C₁₋₂₀ alkyl or aromatic-substituted alkyl of less than 30 carbon atoms;
with the proviso that R⁶ and R⁷ can not both be hydrogen atoms.

36. A 5' -modified oligonucleotide according to claim 35 wherein each R⁶ and R⁷ individually represents a hydrogen atom or phenyl optionally substituted with a C₁₋₈ alkyl, C₁₋₈ alkoxy, 1 to 4 halogen atoms each of atomic number 9 to 35, nitro-, or phenyl, with the proviso that both R⁶ and R⁷ can not both be hydrogen atoms.

37. A 5' -modified oligonucleotide according to claim 35 or 36 wherein R⁶ is a hydrogen atom and R⁷ represents a phenyl, p-butylphenyl, p-methoxyphenyl, p-t-butylphenyl or biphenyl group,

38. A 5' -modified oligonucleotide according to claim 30 or 31 wherein G is-(CH₂)₆-, and Z has the formula:
-S-S-R⁵-O-Y³
where R⁵ is -(CH₂)₆- and Y³ is dimethoxytrityl.

39. A 5' -modified oligonucleotide according to claim 32 or 34 wherein Z has been oxidised to form a terminal aldehyde group on the oligonucleotide.

40. A 5' -modified oligonucleotide according to claim 38 wherein Z has been reduced to form a terminal thiol group on the oligonucleotide,

41. A conjugate of an amino group-containing carrier molecule and a 5' -modified oligonucleotide according to claim 39, wherein the conjugate is formed by reaction between said amino group and said terminal aldehyde group.

42. A conjugate of a carrier molecule having a functional group and a 5'-modified oligonucleotide according to claim 40, wherein the conjugate is formed by reaction between said functional group and said terminal thiol group.

43. A conjugate according to claim 41 or 42 wherein the carrier molecule is a polymer.

44. A conjugate according to claim 43 wherein the polymer is an amino acid polymer.

45. A partially protected alcohol of the formula: 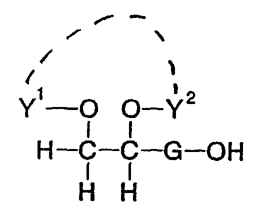
where each Y¹ and Y² represents a hydroxy protecting group or are together joined by a single-atom bridge, other than a -C(=O)- bridging group, to form a five-membered ring protecting group and G represents a C₂₋₂₀ hydrocarbylene group.

46. An alcohol according to claim 45 wherein both Y¹ and Y² are benzoyl and G is butylene.

47. An alcohol according to claim 45 wherein Y¹ is dimethoxytrityl, Y² is benzoyl and G is butylene.

48. An alcohol according to claim 45 wherein each Y¹ and Y² individually represents: 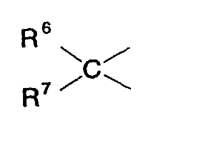
wherein each R⁶ and R⁷ individually represents a hydrogen atom, C₆₋₃₀ aryl or substituted aryl, C₁₋₂₀ alkyl or an aromatic-substituted alkyl group of less than 30 carbon atoms, with the proviso that both R⁶ and R⁷ can not both be hydrogen atoms,

49. A disulfide of the formula
Y³-O-G-S-S-G-OH
wherein G represents a C₄₋₂₀ hydrocarbylene group and Y³ represents a hydroxyl protecting group.

50. A disulfide according to claim 49 wherein Y³ is dimethoxytrityl and G is hexylene.

## Patentansprüche (Patentansprüche für folgende(n) Vertragsstaat(en): AT, BE, CH, LI, DE, DK, FR, GB, IT, LU, MC, NL, PT, SE)

1. Verbindung der Formel: 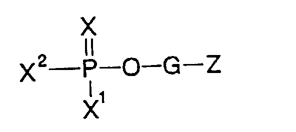 in der
X die folgende Bedeutung hat:
(i) ein Sauerstoff- oder Schwefelatom, wenn X¹ die Gruppe O⁻, Methyl oder -OCH₃ darstellt und X² ein Wasserstoffatom oder den Rest RO- bedeutet, wobei R eine Schutzgruppe darstellt;
(ii) es ist nicht vorhanden, wenn
(a) X¹ ein Chloratom darstellt und X² eine Methylgruppe oder den Rest RO- bedeutet; oder
(b) X² den Rest RO- bedeutet und X¹ den Rest NR¹R² darstellt, wobei jeder der Reste R¹ und R² einzeln einen C₁₋₆-Alkyl-, C₃₋₈-Cycloalkyl- oder einen C₆₋₂₀-Arylrest darstellt oder, wenn sie verbunden sind, mit dem Stickstoffatom eine C₄₋₇-cyclische Struktur bilden mit 0 oder 1 Sauerstoff- oder Schwefelatom;
G einen C₁₋₂₀-Kohlenwasserstoffrest darstellt; und
Z eine hydroxygeschützte vicinale Diolgruppe bedeutet, die über eines der vicinalen Diolkohlenstoffatome an G gebunden ist, oder eine Disulfidgruppe, die über eines der Schwefelatome der Disulfidgruppe an G gebunden ist;
mit der Maßgabe, daß G mindestens 4 Kohlenstoffatome aufweist, wenn Z die Disulfidgruppe bedeutet.

2. Verbindung der Formel 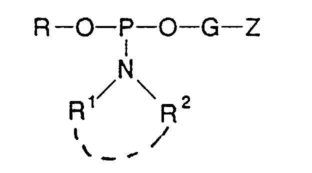
in der R eine Methylgruppe oder eine basenempfindliche Schutzgruppe darstellt;
jeder der Reste R¹ und R² einzeln einen C₁₋₆-Alkyl-, C₃₋₈-Cycloalkyl- oder einen C₆₋₂₀-Arylrest bedeutet, oder, wenn sie verbunden sind, mit dem Stickstoffatom eine C₄₋₇-cyclische Struktur bilden mit 0 oder 1 Sauerstoff- oder Schwefelatom,
G einen C₁₋₂₀-Kohlenwasserstoffrest darstellt; und
Z eine hydroxygeschützte vicinale Diolgruppe bedeutet, die über eines der vicinalen Diolkohlenstoffatome an G gebunden ist, oder eine Disulfidgruppe, die über eines der Schwefelatome der Disulfidgruppe an G gebunden ist;
mit der Maßgabe, daß G mindestens 4 Kohlenstoffatome aufweist, wenn Z die Disulfidgruppe darstellt.

3. Verbindung nach Anspruch 1 oder 2, wobei Z die Formel hat 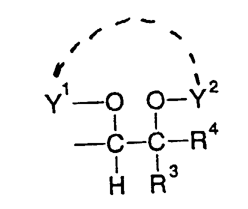
in der jeder der Reste R³ und R⁴ einzeln ein Wasserstoffatom, einen C₁₋₂₀-Alkyl- oder C₆₋₂₀-monocyclischen Arylrest darstellt und jeder der Reste Y¹ und Y² einzeln eine Hydroxyschutzgruppe darstellt oder zusammen über eine Einzelatombrücke verbunden eine 5-gliedrige Ringschutzgruppe bilden;
oder Z den Rest
-S-S-R⁵-O-Y³
darstellt,
wobei R⁵ einen C₁₋₂₀-Alkylenrest oder einen C₆₋₂₀-monocyclischen Arylenrest darstellt und Y³ eine Hydroxyschutzgruppe ist.

4. Verbindung nach Anspruch 3, wobei R⁵ einen C₄₋₆-Alkylenrest darstellt, -OY³ an das ω-Atom des Alkylenrests gebunden ist und Y³ eine Tritylgruppe bedeutet.

5. Verbindung nach einem der vorangehenden Ansprüche, wobei X¹ den Rest NR¹R² darstellt und R¹ und R² jeweils Isopropylgruppen sind.

6. Verbindung nach Anspruch 3 oder 5, wobei R³ und R⁴ beide Wasserstoffatome sind, R⁵ eine -(CH₂)₆-Gruppe ist und Y¹, Y² und Y³ alle Benzoyl- oder Dimethoxytritylgruppen bedeuten.

7. Verbindung nach einem der vorangehenden Ansprüche, wobei X¹ den Rest NR¹R² darstellt und R¹ und R² jeweils Isopropylgruppen sind, G eine -(CH₂)₄-Gruppe bedeutet und entweder R eine β-Cyanoethylgruppe bedeutet oder Z die Formel hat: 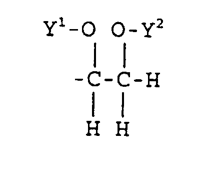
in der Y¹ und Y² jeweils Benzoylgruppen darstellen oder Y¹ eine Benzoylgruppe und Y² eine Dimethoxytritylgruppe ist.

8. Verbindung nach Anspruch 1 oder 2, wobei Z eine hydroxygeschützte vicinale Diolgruppe der Formel bedeutet: 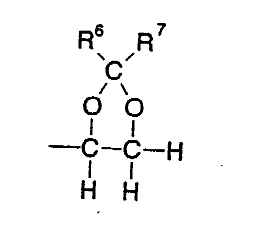
in der jeder der Reste R⁶ und R⁷ einzeln ein Wasserstoffatom, einen C₆₋₃₀-Aryl- oder substituierten Aryl-, C₁₋₂₀-Alkyl- oder einen aromatisch substituierten Alkylrest von weniger als 30 Kohlenstoffatomen darstellt,
mit der Maßgabe, daß R⁶ und R⁷ nicht beide Wasserstoffatome sein können.

9. Verbindung nach Anspruch 8, wobei jeder der Reste R⁶ und R⁷ einzeln ein Wasserstoffatom oder eine Phenylgruppe darstellt, die gegebenenfalls mit C₁₋₈-Alkyl-, C₁₋₈-Alkoxyresten, 1 bis 4 Halogenatomen, die jeweils eine Atomzahl von 9 bis 35 haben, Nitro- oder Phenylgruppen substituiert ist, mit der Maßgabe, daß R⁶ und R⁷ nicht beide Wasserstoffatome sein können.

10. Verbindung nach Anspruch 8 oder 9, wobei R⁶ ein Wasserstoffatom darstellt und R⁷ eine Phenyl-, p-Butylphenyl-, p-Methoxyphenyl-, p-t-Butylphenyl- oder Biphenylgruppe bedeutet.

11. Verbindung nach Anspruch 1 oder 2, wobei X¹ den Rest NR¹R² darstellt und beide Reste R¹ und R² Isopropylgruppen sind, G eine -(CH₂)₆-Gruppe bedeutet und Z die folgende Formel hat:
-S-S-R⁵-O-Y³
in der R⁵ eine -(CH₂)₆-Gruppe bedeutet und Y³ eine Dimethoxytritylgruppe ist.

12. 5'-modifiziertes Oligonucleotid der Formel 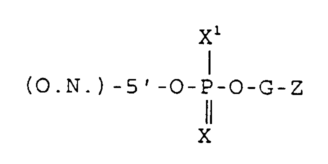
in der (O.N.) eine Oligonucleotidkette darstellt,
X ein Sauerstoff- oder Schwefelatom ist,
X¹ einen O⁻-, Methyl-, -OCH₃- oder NR¹R²-Rest darstellt, wobei jeder der Reste R¹ und R² einzeln ein Wasserstoffatom oder einen C₁₋₆-Alkyl-, C₃₋₈-Cycloalkyl- oder C₆₋₂₀-Arylrest bedeutet, oder, wenn sie verbunden sind, mit dem Stickstoffatom eine C₄₋₇-cyclische Struktur bilden, die keines oder ein Sauerstoff- oder Schwefelatom enthält, G einen C₁₋₂₀-Kohlenwasserstoffrest bedeutet, und
Z eine hydroxygeschützte vicinale Diolgruppe darstellt, die über eines der vicinalen Diolkohlenstoffatome an G gebunden ist, oder eine Disulfidgruppe, die über eines der Schwefelatome der Disulfidgruppe an G gebunden ist.

13. 5'-modifiziertes Oligonucleotid nach Anspruch 12, wobei Z die Formel hat: 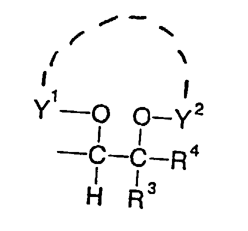
in der jeder der Reste R³ und R⁴ einzeln ein Wasserstoffatom, einen C₄₋₂₀-Alkyl- oder C₆₋₂₀-monocyclischen Arylrest bedeutet und jeder der Reste Y¹ und Y² einzeln eine Hydroxyschutzgruppe bedeutet oder zusammen über eine Einzelatombrücke verbunden eine 5-gliedrige Ringschutzgruppe bilden, oder den Rest
-S-S-R⁵-O-Y³,
bedeutet, wobei R⁵ einen C₁₋₂₀-Alkylenrest oder einen C₆₋₂₀-monocyclischen Arylenrest darstellt und Y³ eine Hydroxyschutzgruppe ist.

14. 5'-modifiziertes Oligonucleotid nach Anspruch 13, wobei Y¹ und Y² entfernt sind, so daß freie Hydroxylgruppen zurückbleiben.

15. 5'-modifiziertes Oligonucleotid nach Anspruch 13, wobei G eine -(CH₂)₄-Gruppe ist und Z die folgende Formel hat: 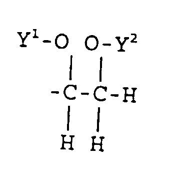
in der Y¹ und Y² beide Benzoylgruppen sind oder Y¹ eine Benzoylgruppe und Y² eine Dimethoxytritylgruppe ist.

16. 5'-modifiziertes Oligonucleotid nach Anspruch 15, wobei Y¹ und Y² entfernt sind, so daß freie Hydroxylgruppen zurückbleiben.

17. 5'-modifiziertes Oligonucleotid nach Anspruch 12 oder 13, wobei Z⁻ die folgende Formel hat: 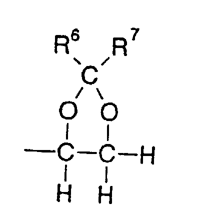
in der jeder der Reste R⁶ und R⁷ einzeln ein Wasserstoffatom, einen C₆₋₃₀-Aryl- oder substituierten Aryl-, C₁₋₂₀-Alkyl- oder aromatisch substituierten Alkylrest von weniger als 30 Kohlenstoffatomen bedeutet,
mit der Maßgabe, daß R⁶ und R⁷ nicht beide Wasserstoffatome sein können.

18. 5'-modifiziertes Oligonucleotid nach Anspruch 17, wobei jeder der Reste R⁶ und R⁷ einzeln ein Wasserstoffatom oder eine Phenylgruppe bedeutet, die gegebenenfalls mit einem C₁₋₈-Alkyl-, C₁₋₈-Alkoxy-, 1 bis 4 Halogenatomen, jeweils mit einer Atomzahl von 9 bis 35, einer Nitro- oder Phenylgruppe substituiert ist, mit der Maßgabe, daß R⁶ und R⁷ nicht beide Wasserstoffatome sein können.

19. 5'-modifiziertes Oligonucleotid nach Anspruch 17 oder 18, wobei R⁶ ein Wasserstoffatom bedeutet und R⁷ eine Phenyl-, p-Butylphenyl-, p-Methoxyphenyl-, p-t-Butylphenyl- oder Biphenylgruppe darstellt.

20. 5'-modifiziertes Oligonucleotid nach Anspruch 12 oder 13, wobei G eine -(CH₂)₆-Gruppe ist und Z die folgende Formel hat:
-S-S-R⁵-O-Y³
in der R⁵ eine -(CH₂)₆-Gruppe ist und Y³ eine Dimethoxytritylgruppe darstellt.

21. 5'-modifiziertes Oligonucleotid nach Anspruch 14 oder 16, wobei Z oxidiert wurde, so daß eine terminale Aldehydgruppe am Oligonucleotid erzeugt wurde.

22. 5'-modifiziertes Oligonucleotid nach Anspruch 20, wobei Z reduziert wurde, so daß eine terminale Thiolgruppe am Oligonucleotid erzeugt wurde.

23. Konjugat eines Aminogruppen tragenden Trägermoleküls und eines 5'-modifizierten Oligonucleotids nach Anspruch 21, wobei das Konjugat durch Umsetzung der Aminogruppe und der terminalen Aldehydgruppe erzeugt wurde.

24. Konjugat eines Trägermoleküls mit einer funktionellen Gruppe und eines 5'-modifizierten Oligonucleotids nach Anspruch 22, wobei das Konjugat durch Umsetzung der funktionellen Gruppe und der terminalen Thiolgruppe erzeugt wurde.

25. Konjugat nach Anspruch 23 oder 24, wobei das Trägermolekül ein Polymer ist.

26. Konjugat nach Anspruch 25, wobei das Polymer ein Aminosäurepolymer ist.

27. Partiell geschützter Alkohol der Formel 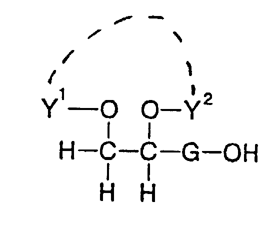
in der jeder der Reste Y¹ und Y² einzeln eine Hydroxyschutzgruppe bedeutet oder miteinander über eine Einzelatombrücke verbunden sind, die keine -C(=O)-Brückengruppe ist, wobei eine 5-gliedrige Ringschutzgruppe erzeugt wird, und G einen C₂₋₂₀-Kohlenwasserstoffrest bedeutet.

28. Alkohol nach Anspruch 27, wobei Y¹ und und Y² beide Benzoylgruppen sind und G eine Butylengruppe ist.

29. Alkohol nach Anspruch 27, wobei Y¹ eine Dimethoxytritylgruppe, Y² eine Benzoylgruppe und G eine Butylengruppe ist.

30. Alkohol nach Anspruch 27, wobei jeder der Reste Y¹ und Y² einzeln den folgenden Rest bedeutet: 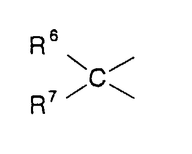
in dem jeder der Reste R⁶ und R⁷ einzeln ein Wasserstoffatom, einen C₆₋₃₀-Aryl- oder substituierten Aryl-, C₁₋₂₀-Alkyl- oder einen aromatisch substituierten Alkylrest von weniger als 30 Kohlenstoffatomen bedeutet, mit der Maßgabe, daß R⁶ und R⁷ nicht beide Wasserstoffatome sein können.

31. Disulfid der Formel
Y³-O-G-S-S-G-OH
in der G einen C₄₋₂₀-Kohlenwasserstoffrest und Y³ eine Hydroxylschutzgruppe bedeutet.

32. Disulfid nach Anspruch 31, wobei Y³ eine Dimethoxytritylgruppe und G eine Hexylengruppe ist.

## Patentansprüche (Patentansprüche für folgende(n) Vertragsstaat(en): ES, GR)

1. Verfahren zur Modifizierung eines Oligonucleotids, umfassend die Umsetzung eines Oligonucleotids mit einer Verbindung der Formel: 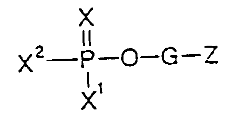 n der
iX die folgende Bedeutung hat:
(i) ein Sauerstoff- oder Schwefelatom, wenn X¹ die Gruppe O⁻, Methyl oder -OCH₃ darstellt und X² ein Wasserstoffatom oder den Rest RO- bedeutet, wobei R eine Schutzgruppe darstellt;
(ii) es ist nicht vorhanden, wenn
(a) X¹ ein Chloratom darstellt und X² eine Methylgruppe oder den Rest RO- bedeutet; oder
(b) X² den Rest RO- bedeutet und X¹ den Rest NR¹R² darstellt, wobei jeder der Reste R¹ und R² einzeln einen C₁₋₆-Alkyl-, C₃₋₈-Cycloalkyl- oder einen C₆₋₂₀-Arylrest darstellt oder, wenn sie verbunden sind, mit dem Stickstoffatom eine C₄₋₇-cyclische Struktur bilden mit 0 oder 1 Sauerstoff- oder Schwefelatom;
G einen C₁₋₂₀-Kohlenwasserstoffrest darstellt; und
Z eine hydroxygeschützte vicinale Diolgruppe bedeutet, die über eines der vicinalen Diolkohlenstoffatome an G gebunden ist, oder eine Disulfidgruppe, die über eines der Schwefelatome der Disulfidgruppe an G gebunden ist;
mit der Maßgabe, daß G mindestens 4 Kohlenstoffatome aufweist, wenn Z die Disulfidgruppe bedeutet,
zur Herstellung eines 5'-modifizierten Oligonucleotids.

2. Verfahren zur Modifizierung eines Oligonucleotids, umfassend die Umsetzung eines Oligonucleotids mit einer Verbindung der Formel 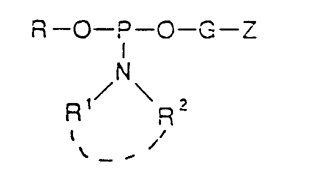
in der R eine Methylgruppe oder eine basenempfindliche Schutzgruppe darstellt;
jeder der Reste R¹ und R² einzeln einen C₁₋₆-Alkyl-, C₃₋₈-Cycloalkyl- oder einen C₆₋₂₀-Arylrest bedeutet, oder, wenn sie verbunden sind, mit dem Stickstoffatom eine C₄₋₇-cyclische Struktur bilden mit 0 oder 1 Sauerstoff- oder Schwefelatom,
G einen C₁₋₂₀-Kohlenwasserstoffrest darstellt; und
Z eine hydroxygeschützte vicinale Diolgruppe bedeutet, die über eines der vicinalen Diolkohlenstoffatome an G gebunden ist, oder eine Disulfidgruppe, die über eines der Schwefelatome der Disulfidgruppe an G gebunden ist;
mit der Maßgabe, daß G mindestens 4 Kohlenstoffatome aufweist, wenn Z die Disulfidgruppe darstellt,
zur Herstellung eines 5'-modifizierten Oligonucleotids.

3. Verfahren nach Anspruch 1 oder 2, wobei Z die Formel hat 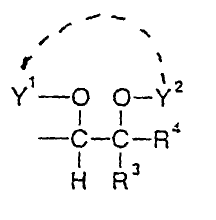
in der jeder der Reste R³ und R⁴ einzeln ein Wasserstoffatom, einen C₁₋₂₀-Alkyl- oder C₆₋₂₀-monocyclischen Arylrest darstellt und jeder der Reste Y¹ und Y² einzeln eine Hydroxyschutzgruppe darstellt oder zusammen über eine Einzelatombrücke verbunden eine 5-gliedrige Ringschutzgruppe bilden;
oder Z den Rest
-S-S-R⁵-O-Y³
darstellt,
wobei R⁵ einen C₁₋₂₀-Alkylenrest oder einen C₆₋₂₀-monocyclischen Arylenrest darstellt und Y³ eine Hydroxyschutzgruppe ist.

4. Verfahren nach Anspruch 3, wobei R⁵ einen C₄₋₆-Alkylenrest darstellt, -OY³ an das ω-Atom des Alkylenrests gebunden ist und Y³ eine Tritylgruppe bedeutet.

5. Verfahren nach einem der vorangehenden Ansprüche, wobei X¹ den Rest NR¹R² darstellt und R¹ und R² jeweils Isopropylgruppen sind.

6. Verfahren nach Anspruch 3 oder 5, wobei R³ und R⁴ beide Wasserstoffatome sind, R⁵ eine -(CH₂)₆-Gruppe ist und Y¹, Y² und Y³ alle Benzoyl- oder Dimethoxytritylgruppen bedeuten.

7. Verfahren nach einem der vorangehenden Ansprüche, wobei X¹ den Rest NR¹R² darstellt und R¹ und R² jeweils Isopropylgruppen sind, G eine -(CH2)4-Gruppe bedeutet und entweder R eine β-Cyanoethylgruppe bedeutet oder Z die Formel hat: 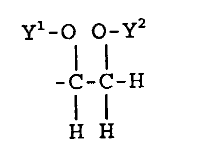
in der Y¹ und Y² jeweils Benzoylgruppen darstellen oder Y¹ eine Benzoylgruppe und Y² eine Dimethoxytritylgruppe ist.

8. Verfahren nach Anspruch 1 oder 2, wobei Z eine hydroxygeschützte vicinale Diolgruppe der Formel bedeutet: 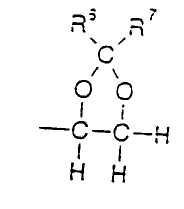
in der jeder der Reste R⁶ und R⁷ einzeln ein Wasserstoffatom, einen C₆₋₃₀-Aryl- oder substituierten Aryl-, C₁₋₂₀-Alkyl- oder einen aromatisch substituierten Alkylrest von weniger als 30 Kohlenstoffatomen darstellt,
mit der Maßgabe, daß R⁶ und R⁷ nicht beide Wasserstoffatome sein können.

9. Verfahren nach Anspruch 8, wobei jeder der Reste R⁶ und R⁷ einzeln ein Wasserstoffatom oder eine Phenylgruppe darstellt, die gegebenenfalls mit C₁₋₈-Alkyl-, C₁₋₈-Alkoxyresten, 1 bis 4 Halogenatomen, die jeweils eine Atomzahl von 9 bis 35 haben, Nitro- oder Phenylgruppen substituiert ist, mit der Maßgabe, daß R⁶ und R⁷ nicht beide Wasserstoffatome sein können.

10. Verfahren nach Anspruch 8 oder 9, wobei R⁶ ein Wasserstoffatom darstellt und R⁷ eine Phenyl-, p-Butylphenyl-, p-Methoxyphenyl-, p-t-Butylphenyl- oder Biphenylgruppe bedeutet.

11. Verfahren nach Anspruch 1 oder 2, wobei X¹ den Rest NR¹R² darstellt und beide Reste R¹ und R² Isopropylgruppen sind, G eine -(CH₂)₆-Gruppe bedeutet und Z die folgende Formel hat:
-S-S-R⁵-O-Y³
in der R⁵ eine -(CH₂)₆-Gruppe bedeutet und Y³ eine Dimethoxytritylgruppe ist.

12. Verfahren nach einem der vorangehenden Ansprüche, ferner umfassend den Schritt der Entfernung der Hydroxyschutzgruppen, so daß freie Hydroxygruppen zurückbleiben.

13. Verfahren nach Anspruch 12, ferner umfassend die Oxidation von Z, so daß eine terminale Aldehydgruppe am Oligonucleotid erzeugt wird.

14. Verfahren nach Anspruch 11, ferner umfassend die Reduktion von Z, so daß eine terminale Thiolgruppe am Oligonucleotid erzeugt wird.

15. Verfahren nach Anspruch 13, ferner umfassend die Konjugation eines Amino-enthaltenden Trägermoleküls mit der terminalen Aldehydgruppe.

16. Verfahren nach Anspruch 14, ferner umfassend die Konjugation eines eine funktionelle Gruppe enthaltenden Trägermoleküls mit der terminalen Thiolgruppe.

17. Verfahren nach Anspruch 15 oder 16, wobei das Trägermolekül ein Polymer ist.

18. Verfahren nach Anspruch 17, wobei das Polymer ein Aminosäurepolymer ist.

19. Verbindung der Formel: 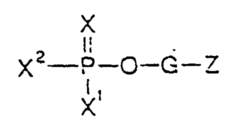 in der
X die folgende Bedeutung hat:
(i) ein Sauerstoff- oder Schwefelatom, wenn X¹ die Gruppe O⁻, Methyl oder -OCH₃ darstellt und X² ein Wasserstoffatom oder den Rest RO- bedeutet, wobei R eine Schutzgruppe darstellt;
(ii) es ist nicht vorhanden, wenn
(a) X¹ ein Chloratom darstellt und X² eine Methylgruppe oder den Rest RO- bedeutet; oder
(b) X² den Rest RO- bedeutet und X¹ den Rest NR¹R² darstellt, wobei jeder der Reste R¹ und R² einzeln einen C₁-₆-Alkyl-, C₃₋₈-Cycloalkyl- oder einen C₆₋₂₀-Arylrest darstellt oder, wenn sie verbunden sind, mit dem Stickstoffatom eine C₄₋₇-cyclische Struktur bilden mit 0 oder 1 Sauerstoff- oder Schwefelatom;
G einen C₁₋₂₀-Kohlenwasserstoffrest darstellt; und
Z eine hydroxygeschützte vicinale Diolgruppe bedeutet, die über eines der vicinalen Diolkohlenstoffatome an G gebunden ist, oder eine Disulfidgruppe, die über eines der Schwefelatome der Disulfidgruppe an G gebunden ist;
mit der Maßgabe, daß G mindestens 4 Kohlenstoffatome aufweist, wenn Z die Disulfidgruppe bedeutet.

20. Verbindung der Formel 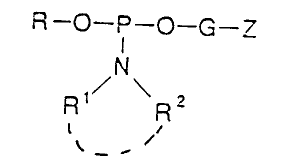
in der R eine Methylgruppe oder eine basenempfindliche Schutzgruppe darstellt;
jeder der Reste R¹ und R² einzeln einen C₁₋₆-Alkyl-, C₃₋₆-Cycloalkyl- oder einen C₆₋₂₀-Arylrest bedeutet, oder, wenn sie verbunden sind, mit dem Stickstoffatom eine C₄₋₇-cyclische Struktur bilden mit 0 oder 1 Sauerstoff- oder Schwefelatom,
G einen C₁₋₂₀-Kohlenwasserstoffrest darstellt; und
Z eine hydroxygeschützte vicinale Diolgruppe bedeutet, die über eines der vicinalen Diolkohlenstoffatome an G gebunden ist, oder eine Disulfidgruppe, die über eines der Schwefelatome der Disulfidgruppe an G gebunden ist;
mit der Maßgabe, daß G mindestens 4 Kohlenstoffatome aufweist, wenn Z die Disulfidgruppe darstellt.

21. Verbindung nach Anspruch 19 oder 20, wobei Z die Formel hat 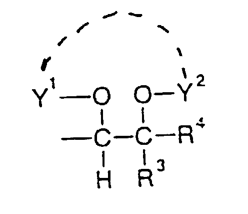
in der jeder der Reste R³ und R⁴ einzeln ein Wasserstoffatom, einen C₁₋₂₀-Alkyl- oder C₆₋₂₀-monocyclischen Arylrest darstellt und jeder der Reste Y¹ und Y² einzeln eine Hydroxyschutzgruppe darstellt oder zusammen über eine Einzelatombrücke verbunden eine 5-gliedrige Ringschutzgruppe bilden;
oder Z den Rest
-S-S-R⁵-O-Y³
darstellt,
wobei R⁵ einen C₁₋₂₀-Alkylenrest oder einen C₆₋₂₀-monocyclischen Arylenrest darstellt und Y³ eine Hydroxyschutzgruppe ist.

22. Verbindung nach Anspruch 21, wobei R⁵ einen C₄₋₆-Alkylenrest darstellt, -OY³ an das ω-Atom des Alkylenrests gebunden ist und Y³ eine Tritylgruppe bedeutet.

23. Verbindung nach einem der Ansprüche 19 bis 22, wobei X¹ den Rest NR¹R² darstellt und R¹ und R² jeweils Isopropylgruppen sind.

24. Verbindung nach Anspruch 21 oder 23, wobei R³ und R⁴ beide Wasserstoffatome sind, R⁵ eine -(CH₂)₆-Gruppe ist und Y¹, Y² und Y³ alle Benzoyl- oder Dimethoxytritylgruppen bedeuten.

25. Verbindung nach einem der Ansprüche 19 bis 24, wobei X¹ den Rest NR¹R² darstellt und R¹ und R² jeweils Isopropylgruppen sind, G eine -(CH₂)₄-Gruppe bedeutet und entweder R eine β-Cyanoethylgruppe bedeutet oder Z die Formel hat: 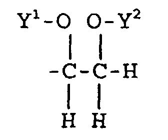
in der Y¹ und Y² jeweils Benzoylgruppen darstellen oder Y¹ eine Benzoylgruppe und Y² eine Dimethoxytritylgruppe ist.

26. Verbindung nach Anspruch 19 oder 20, wobei Z eine hydroxygeschützte vicinale Diolgruppe der Formel bedeutet: 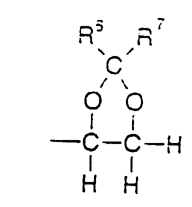
in der jeder der Reste R⁶ und R⁷ einzeln ein Wasserstoffatom, einen C₆₋₃₀-Aryl- oder substituierten Aryl-, C₁₋₂₀-Alkyl- oder einen aromatisch substituierten Alkylrest von weniger als 30 Kohlenstoffatomen darstellt,
mit der Maßgabe, daß R⁶ und R⁷ nicht beide Wasserstoffatome sein können.

27. Verbindung nach Anspruch 26, wobei jeder der Reste R⁶ und R⁷ einzeln ein Wasserstoffatom oder eine Phenylgruppe darstellt, die gegebenenfalls mit C₁₋₈-Alkyl-, C₁₋₈-Alkoxyresten, 1 bis 4 Halogenatomen, die jeweils eine Atomzahl von 9 bis 35 haben, Nitro- oder Phenylgruppen substituiert ist, mit der Maßgabe, daß R⁶ und R⁷ nicht beide Wasserstoffatome sein können.

28. Verbindung nach Anspruch 27 oder 28, wobei R⁶ ein Wasserstoffatom darstellt und R⁷ eine Phenyl-, p-Butylphenyl-, p-Methoxyphenyl-, p-t-Butylphenyl- oder Biphenylgruppe bedeutet.

29. Verbindung nach Anspruch 19 oder 20, wobei X¹ den Rest NR¹R² darstellt und beide Reste R¹ und R² Isopropylgruppen sind, G eine -(CH₂)₆-Gruppe bedeutet und Z die folgende Formel hat:
-S-S-R⁵-O-Y³
in der R⁵ eine -(CH₂)₆-Gruppe bedeutet und Y³ eine Dimethoxytritylgruppe ist.

30. 5'-modifiziertes Oligonucleotid der Formel 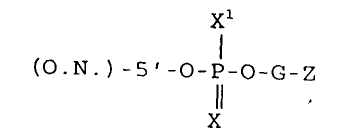
in der (O.N.) eine Oligonucleotidkette darstellt,
X ein Sauerstoff- oder Schwefelatom ist,
X¹ einen O⁻-, Methyl-, -OCH3- oder NR¹R²-Rest darstellt, wobei jeder der Reste R¹ und R² einzeln ein Wasserstoffatom oder einen C₁₋₆-Alkyl-, C₃₋₈-Cycloalkyl- oder C₆₋₂₀-Arylrest bedeutet, oder, wenn sie verbunden sind, mit dem Stickstoffatom eine C₄₋₇-cyclische Struktur bilden, die keines oder ein Sauerstoff- oder Schwefelatom enthält, G einen C₁₋₂₀-Kohlenwasserstoffrest bedeutet, und
Z eine hydroxygeschützte vicinale Diolgruppe darstellt, die über eines der vicinalen Diolkohlenstoffatome an G gebunden ist, oder eine Disulfidgruppe, die über eines der Schwefelatome der Disulfidgruppe an G gebunden ist.

31. 5'-modifiziertes Oligonucleotid nach Anspruch 30, wobei Z die Formel hat: 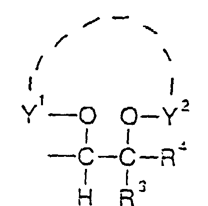
in der jeder der Reste R³ und R⁴ einzeln ein Wasserstoffatom, einen C₄₋₂₀-Alkyl- oder C₆₋₂₀-monocyclischen Arylrest bedeutet und jeder der Reste Y¹ und Y² einzeln eine Hydroxyschutzgruppe bedeutet oder zusammen über eine Einzelatombrücke verbunden eine 5-gliedrige Ringschutzgruppe bilden, oder den Rest
-S-S-R⁵-O-Y³,
bedeutet, wobei R⁵ einen C₁₋₂₀-Alkylenrest oder einen C₆₋₂₀-monocyclischen Arylenrest darstellt und Y³ eine Hydroxyschutzgruppe ist.

32. 5'-modifiziertes Oligonucleotid nach Anspruch 31, wobei Y¹ und Y² entfernt sind, so daß freie Hydroxylgruppen zurückbleiben.

33. 5'-modifiziertes Oligonucleotid nach Anspruch 31, wobei G eine -(CH₂)₄-Gruppe ist und Z die folgende Formel hat: 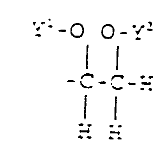
in der Y¹ und Y² beide Benzoylgruppen sind oder Y¹ eine Benzoylgruppe und Y² eine Dimethoxytritylgruppe ist.

34. 5'-modifiziertes Oligonucleotid nach Anspruch 33, wobei Y¹ und Y² entfernt sind, so daß freie Hydroxylgruppen zurückbleiben.

35. 5'-modifiziertes Oligonucleotid nach Anspruch 30 oder 31, wobei Z die folgende Formel hat: 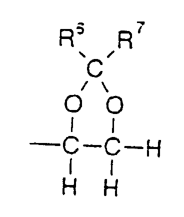
in der jeder der Reste R⁶ und R⁷ einzeln ein Wasserstoffatom, einen C₆₋₃₀-Aryl- oder substituierten Aryl-, C₁₋₂₀-Alkyl- oder aromatisch substituierten Alkylrest von weniger als 30 Kohlenstoffatomen bedeutet,
mit der Maßgabe, daß R⁶ und R⁷ nicht beide Wasserstoffatome sein können.

36. 5'-modifiziertes Oligonucleotid nach Anspruch 35, wobei jeder der Reste R⁶ und R⁷ einzeln ein Wasserstoffatom oder eine Phenylgruppe bedeutet, die gegebenenfalls mit einem C₁₋₈-Alkyl-, C₁₋₈-Alkoxy-, 1 bis 4 Halogenatomen, jeweils mit einer Atomzahl von 9 bis 35, einer Nitro- oder Phenylgruppe substituiert ist, mit der Maßgabe, daß R⁶ und R⁷ nicht beide Wasserstoffatome sein können.

37. 5'-modifiziertes Oligonucleotid nach Anspruch 35 oder 36, wobei R⁶ ein Wasserstoffatom bedeutet und R⁷ eine Phenyl-, p-Butylphenyl-, p-Methoxyphenyl-, p-t-Butylphenyl- oder Biphenylgruppe darstellt.

38. 5'-modifiziertes Oligonucleotid nach Anspruch 30 oder 31, wobei G eine -(CH₂)₆-Gruppe ist und Z die folgende Formel hat:
-S-S-R⁵-O-Y³
in der R⁵ eine -(CH₂)₆-Gruppe ist und Y³ eine Dimethoxytritylgruppe darstellt.

39. 5'-modifiziertes Oligonucleotid nach Anspruch 32 oder 34, wobei Z oxidiert wurde, so daß eine terminale Aldehydgruppe am Oligonucleotid erzeugt wurde.

40. 5'-modifiziertes Oligonucleotid nach Anspruch 38, wobei Z reduziert wurde, so daß eine terminale Thiolgruppe am Oligonucleotid erzeugt wurde.

41. Konjugat eines Aminogruppen tragenden Trägermoleküls und eines 5'-modifizierten Oligonucleotids nach Anspruch 39, wobei das Konjugat durch Umsetzung der Aminogruppe und der terminalen Aldehydgruppe erzeugt wurde.

42. Konjugat eines Trägermoleküls mit einer funktionellen Gruppe und eines 5'-modifizierten Oligonucleotids nach Anspruch 40, wobei das Konjugat durch Umsetzung der funktionellen Gruppe und der terminalen Thiolgruppe erzeugt wurde.

43. Konjugat nach Anspruch 41 oder 42, wobei das Trägermolekül ein Polymer ist.

44. Konjugat nach Anspruch 43, wobei das Polymer ein Aminosäurepolymer ist.

45. Partiell geschützter Alkohol der Formel 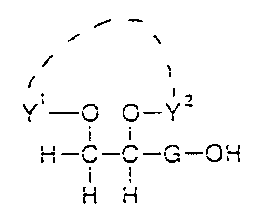
in der jeder der Reste Y¹ und Y² einzeln eine Hydroxyschutzgruppe bedeutet oder miteinander über eine Einzelatombrücke verbunden sind, die keine -C(=O)-Brückengruppe ist, wobei eine 5-gliedrige Ringschutzgruppe erzeugt wird, und G einen C₂₋₂₀-Kohlenwasserstoffrest bedeutet.

46. Alkohol nach Anspruch 45, wobei Y¹ und und Y² beide Benzoylgruppen sind und G eine Butylengruppe ist.

47. Alkohol nach Anspruch 45, wobei Y¹ eine Dimethoxytritylgruppe, Y² eine Benzoylgruppe und G eine Butylengruppe ist.

48. Alkohol nach Anspruch 45, wobei jeder der Reste Y¹ und Y² einzeln den folgenden Rest bedeutet: 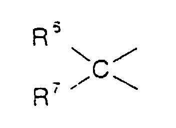
in dem jeder der Reste R⁶ und R⁷ einzeln ein Wasserstoffatom, einen C₆₋₃₀-Aryl- oder substituierten Aryl-, C₁₋₂₀-Alkyl- oder einen aromatisch substituierten Alkylrest von weniger als 30 Kohlenstoffatomen bedeutet, mit der Maßgabe, daß R⁶ und R⁷ nicht beide Wasserstoffatome sein können.

49. Disulfid der Formel
Y³-O-G-S-S-G-OH
in der G einen C₄₋₂₀-Kohlenwasserstoffrest und Y³ eine Hydroxylschutzgruppe bedeutet.

50. Disulfid nach Anspruch 49, wobei Y³ eine Dimethoxytritylgruppe und G eine Hexylengruppe ist.

## Revendications (Revendications pour l'(les) Etat(s) contractant(s) suivant(s): AT, BE, CH, LI, DE, DK, FR, GB, IT, LU, MC, NL, PT, SE)

1. Composé de formule : 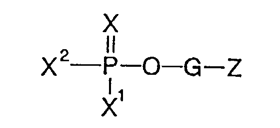 dans laquelle
X est :
(i) un atome d'oxygène ou de soufre lorsque X¹ représente O⁻, un méthyle ou -OCH₃ et X² représente un atome d'hydrogène ou RO-, dans laquelle R représente un groupe protecteur ;
(ii) non présent lorsque
(a) X¹ représente un atome de chlore et X² représente un méthyle ou RO- ; ou
(b) X² représente RO- et X¹ représente NR¹R² dans laquelle chaque R¹ et R² représentent individuellement un groupe alkyle en C₁-C₆, cycloalkyle en C₃-C₈ ou aryle en C₆-C₂₀ ou, lorsqu'ils sont attachés ensemble, forment avec l'atome d'azote une structure cyclique en C₄-C₇ avec 0 ou 1 atome d'oxygène ou de soufre ;
G représente un groupe hydrocarbylène en C₁-C₂₀ ; et
Z représente un groupe diol adjacent à hydroxy protégé, lié à G par l'un des atomes de carbone du diol voisin ou un groupe disulfure lié à G par l'un des atomes de soufre du groupe disulfure ;
à la condition que G ait au moins 4 atomes de carbone lorsque Z représente ledit groupe disulfure.

2. Composé de formule : 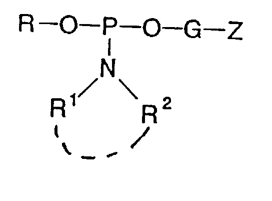
dans laquelle R représente un groupe méthyle ou un groupe protecteur sensible aux bases ;
chaque R¹ et R² représente individuellement un groupe alkyle en C₁-C₆, cycloalkyle en C₃-C₈, ou aryle en C₆-C₂₀ ou, lorsqu'ils sont attachés ensemble, forment avec l'atome d'azote une structure cyclique en C₄-C₇ ayant 0 ou 1 atome d'oxygène ou de soufre ;
G représente un groupe hydrocarbylène en C₁-C₂₀ ; et
Z représente un groupe diol voisin, à hydroxy protégé, lié à G par l'un des atomes de carbone du diol voisin ou un groupe disulfure lié à G par l'un des atomes de soufre du groupe disulfure ;
à la condition que G ait au moins 4 atomes de carbone lorsque Z représente ledit groupe disulfure.

3. Composé selon la revendication 1 ou 2 dans lequel Z a la formule : 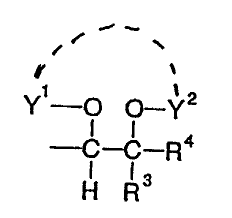
dans laquelle chaque R³ et R⁴ représentent individuellement un atome d'hydrogène, un groupe alkyle en C₁-C₂₀ ou aryle monocyclique en C₆-C₂₀, et chaque Y¹ et Y² représentent individuellement un groupe protecteur d'hydroxy ou sont attachés ensemble par un pont monoatomique pour former un groupe protecteur cyclique à cinq membres ;
ou Z représente
-S-S-R⁵-O-Y³
dans lequel R⁵ représente un groupe alkylène en C₁-C₂₀ ou un groupe arylène monocyclique en C₆-C₂₀ et Y³ est un groupe protecteur d'hydroxy.

4. Composé selon la revendication 3 dans lequel R⁵ représente un groupe alkylène en C₄-C₆, -OY³ est lié à l'atome ω du groupe alkylène et Y³ est un trityle.

5. Composé selon l'une quelconque des revendications précédentes dans lequel X¹ représente NR¹R² et R¹ et R² sont tous deux un isopropyle.

6. Composé selon la revendication 3 ou 5 dans lequel R³ et R⁴ sont tous deux un hydrogène, R⁵ est -(CH₂)₆, et Y¹, Y² et Y³ sont tous un benzoyle ou un diméthoxytrityle.

7. Composé selon l'une quelconque des revendications précédentes dans lequel X¹ représente NR¹R² et R¹ et R² sont tous deux un isopropyle, G est -(CH₂)₄, et soit R est un β-cyanoéthyle soit Z a la formule : 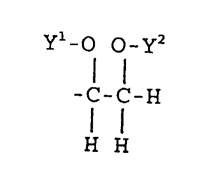
dans laquelle Y¹ et Y² sont tous deux un benzoyle ou Y¹ est un benzoyle et Y² est un diméthoxytrityle.

8. Composé selon la revendication 1 ou 2 dans lequel Z représente un groupe diol voisin à hydroxy protégé de formule : 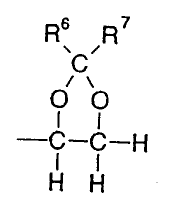
dans laquelle chaque R⁶ et R⁷ représente individuellement un atome d'hydrogène, un groupe aryle en C₆-C₃₀ ou aryle substitué, alkyle en C₁-C₂₀ ou alkyle de moins de 30 atomes de carbone substitué par des groupes aromatiques ;
à la condition que R⁶ et R⁷ ne soient pas tous deux des atomes d'hydrogène.

9. Composé selon la revendication 8 dans lequel chaque R⁶ et R⁷ représente individuellement un atome d'hydrogène ou un phényle éventuellement substitué par un alkyle en C₁-C₈, un alkoxy en C₁-C₈, 1 à 4 atomes d'halogène chacun de nombre atomique compris entre 9 et 35, un nitro, ou un phényle, à la condition que R⁶ et R⁷ ne soient pas tous deux des atomes d'hydrogène.

10. Composé selon la revendication 8 ou 9 dans lequel R⁶ représente un atome d'hydrogène et R⁷ représente un groupe phényle, p-butylphényle, p-méthoxyphényle, p-t-butylphényle ou biphényle.

11. Composé selon la revendication 1 ou 2 dans lequel X¹ représente NR¹R² et R¹ et R² sont tous deux un isopropyle, G est -(CH₂)₆-, et Z a la formule :
-S-S-R⁵-O-Y³
dans laquelle R⁵ est -(CH₂)₆- et Y³ est un diméthoxytrityle.

12. Oligonucléotide modifié en 5' de formule : 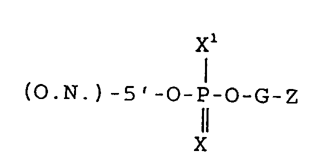
dans laquelle (O.N.) représente une chaîne oligonucléotidique ;
X est un atome d'oxygène ou de soufre ;
X¹ représente O⁻, un méthyle, -OCH₃ ou NR¹R² dans lequel chaque R¹ et R² représente individuellement un atome d'hydrogène ou un groupe alkyle en C₁-C₆, cycloalkyle en C₃-C₈ ou aryle en C₆-C₂₀ ou, lorsqu'ils sont attachés ensemble, forment avec l'atome d'azote une structure cyclique en C₄-C₇ contenant aucun ou un atome d'oxygène ou de soufre ;
G est un groupe hydroxycarbylène en C₁-C₂₀ ; et
Z est un groupe diol voisin, à hydroxy protégé, lié à G par l'un des atomes de carbone du diol voisin ou un groupe disulfure lié à G par l'un des atomes de soufre du groupe disulfure.

13. Oligonucléotide modifié en 5' selon la revendication 12 dans lequel Z a la formule : 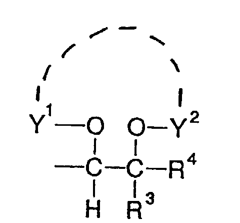
dans laquelle chaque R³ et R⁴ représentent individuellement un atome d'hydrogène, un groupe alkyle en C₄-C₂₀ ou aryle monocyclique en C₆-C₂₀ et chaque Y¹ et Y² représente individuellement un groupe protecteur d'hydroxy ou sont attachés ensemble par un pont monoatomique pour former un groupe protecteur cyclique à cinq membres, ou
-S-S-R⁵-O-Y³
dans lequel R⁵ représente un groupe alkylène en C₁-C₂₀ ou un groupe arylène monocyclique en C₆-C₂₀ et Y³ est un groupe protecteur d'hydroxy.

14. Oligonucléotide modifié en 5' selon la revendication 13 dans lequel Y¹ et Y² sont éliminés pour laisser des groupes hydroxy libres.

15. Oligonucléotide modifié en 5' selon la revendication 13 dans lequel G est -(CH₂)₄-, et Z a la formule : 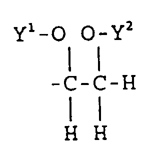
dans laquelle Y¹ et Y² sont tous deux un benzoyle ou Y¹ est un benzoyle et Y² est un diméthoxytrityle.

16. Oligonucléotide modifié en 5' selon la revendication 15 dans lequel Y¹ et Y² sont éliminés pour laisser des groupes hydroxy libres.

17. Oligonucléotide modifié en 5' selon la revendication 12 ou 13 dans lequel Z a la formule : 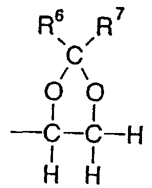
dans laquelle chaque R⁶ et R⁷ représentent individuellement un atome d'hydrogène, un aryle en C₆-C₃₀ ou un aryle substitué, un alkyle en C₁-C₂₀ ou un alkyle de moins de 30 atomes de carbone substitué par des groupes aromatiques ;
à la condition que R⁶ et R⁷ ne soient pas tous deux des atomes d'hydrogène.

18. Oligonucléotide modifié en 5' selon la revendication 17 dans lequel chaque R⁶ et R⁷ représente individuellement un atome d'hydrogène ou un phényle éventuellement substitué par un alkyle en C₁-C₈, un alkoxy en C₁-C₈, 1 à 4 atomes d'halogène chacun de nombre atomique compris entre 9 et 35, un nitro, ou un phényle, à la condition que R⁶ et R⁷ ne soient pas tous deux des atomes d'hydrogène.

19. Oligonucléotide modifié en 5' selon la revendication 17 ou 18 dans lequel R⁶ est un atome d'hydrogène et R⁷ représente un groupe phényle, p-butylphényle, p-méthoxyphényle, p-t-butylphényle ou biphényle.

20. Oligonucléotide modifié en 5' selon la revendication 12 ou 13 dans lequel G est -(CH₂)₆- et Z a la formule :
-S-S-R⁵-O-Y³
dans laquelle R⁵ est -(CH₂)₆- et Y³ est un diméthoxytrityle.

21. Oligonucléotide modifié en 5' selon la revendication 14 ou 16 dans lequel Z a été oxydé pour former un groupe aldéhyde terminal sur l'oligonucléotide.

22. Oligonucléotide modifié en 5' selon la revendication 20 dans lequel Z a été réduit pour former un groupe thiol terminal sur l'oligonucléotide.

23. Conjugué d'une molécule porteuse contenant un groupe amino et d'un oligonucléotide modifié en 5' selon la revendication 21, dans lequel le conjugué est formé par réaction entre ledit groupe amino et ledit groupe aldéhyde terminal.

24. Conjugué d'une molécule porteuse ayant un groupe fonctionnel et un oligonucléotide modifié en 5' selon la revendication 22, dans lequel le conjugué est formé par réaction entre ledit groupe fonctionnel et ledit groupe thiol terminal.

25. Conjugué selon la revendication 23 ou 24 dans lequel la molécule porteuse est un polymère.

26. Conjugué selon la revendication 25 dans lequel le polymère est un polymère d'acides aminés.

27. Alcool partiellement protégé de formule : 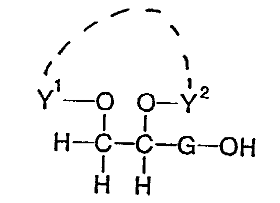
dans lequel chaque Y¹ et Y² représente individuellement un groupe protecteur d'hydroxy ou sont attachés ensemble par un pont monoatomique autre qu'un groupe de pontage -C(=O)-, pour former un groupe protecteur cyclique à cinq membres et G représente un groupe hydroxycarbylène en C₂-C₂₀.

28. Alcool selon la revendication 27 dans lequel Y¹ et Y² sont tous deux un benzoyle et G est un butylène.

29. Alcool selon la revendication 27 dans lequel Y¹ est un diméthoxytrityle, Y² est un benzoyle et G est un butylène.

30. Alcool selon la revendication 27 dans lequel chaque Y¹ et Y² représentent individuellement : 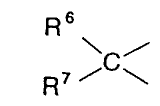
dans lequel chaque R⁶ et R⁷ représentent individuellement un atome d'hydrogène, un groupe aryle en C₆-C₃₀ ou aryle substitué, alkyle en C₁-C₂₀ ou alkyle de moins de 30 atomes de carbone substitué par des groupes aromatiques à la condition que R⁶ et R⁷ ne soient pas tous deux des atomes d'hydrogène.

31. Disulfure de formule
Y³-O-G-S-S-G-OH
dans laquelle G représente un groupe hydrocarbylène en C₄-C₂₀ et Y³ représente un groupe protecteur d'hydroxyle.

32. Disulfure selon la revendication 31 dans lequel Y³ est un diméthoxytrityle et G est un hexylène.

## Revendications (Revendications pour l'(les) Etat(s) contractant(s) suivant(s): ES, GR)

1. Procédé de modification d'un oligonucléotide comprenant la réaction d'un oligonucléotide avec un composé de formule : 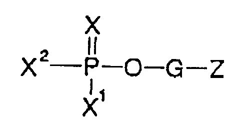 dans laquelle
X est :
(i) un atome d'oxygène ou de soufre lorsque X¹ représente O⁻, un méthyle ou -OCH₃ et X² représente un atome d'hydrogène ou RO-, dans laquelle R représente un groupe protecteur ;
(ii) non présent lorsque
(a) X¹ représente un atome de chlore et X² représente un méthyle ou RO- ; ou
(b) X² représente RO- et X¹ représente NR¹R² dans laquelle chaque R¹ et R² représentent individuellement un groupe alkyle en C₁-C₆, cycloalkyle en C₃-C₈ ou aryle en C₆-C₂₀ ou, lorsqu'ils sont attachés ensemble, forment avec l'atome d'azote une structure cyclique en C₄-C₇ avec 0 ou 1 atome d'oxygène ou de soufre ;
G représente un groupe hydrocarbylène en C₁-C₂₀ ; et
Z représente un groupe diol voisin à hydroxy protégé, lié à G par l'un des atomes de carbone du diol ou un groupe disulfure lié à G par l'un des atomes de soufre du groupe disulfure ;
à la condition que G ait au moins 4 atomes de carbone lorsque Z représente ledit groupe disulfure ;
pour produire un nucléotide modifié en 5'.

2. Procédé de modification d'un nucléotide comprenant la réaction d'un oligonucléotide avec un composé de formule : 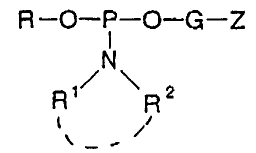
dans laquelle R représente un groupe méthyle ou un groupe protecteur sensible aux bases ;
chaque R¹ et R² représentent individuellement un groupe alkyle en C₁-C₆, cycloalkyle en C₃-C₈, ou aryle en C₆-C₂₀ ou, lorsqu'ils sont attachés ensemble, forment avec l'atome d'azote une structure cyclique en C₄-C₇ ayant 0 ou 1 atome d'oxygène ou de soufre ;
G représente un groupe hydrocarbylène en C₁-C₂₀ ; et
Z représente un groupe diol voisin à hydroxy protégé lié à G par l'un des atomes de carbone du diol voisin ou un groupe disulfure lié à G par l'un des atomes de soufre du groupe disulfure ;
à la condition que G ait au moins 4 atomes de carbone lorsque Z représente ledit groupe disulfure ;
pour produire un nucléotide modifié en 5'.

3. Procédé selon la revendication 1 ou 2 dans lequel Z a la formule : 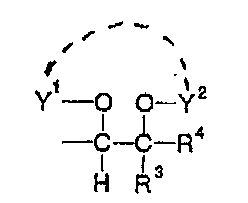
dans laquelle chaque R³ et R⁴ représentent individuellement un atome d'hydrogène, un groupe alkyle en C₁-C₂₀ ou aryle monocyclique en C₆-C₂₀, et chaque Y¹ et Y² représentent individuellement un groupe protecteur d'hydroxy ou sont attachés ensemble par un pont monoatomique pour former un groupe protecteur cyclique à cinq membres ;
ou Z représente
-S-S-R⁵-O-Y³
dans lequel R⁵ représente un groupe alkylène en C₁-C₂₀ ou un groupe arylène monocyclique en C₆-C₂₀ et Y³ est un groupe protecteur d'hydroxy.

4. Procédé selon la revendication 3 dans lequel R⁵ représente un groupe alkylène en C₄-C₆, -OY³ est lié à l'atome ω du groupe alkylène et Y³ est un trityle.

5. Procédé selon l'une quelconque des revendications précédentes dans lequel X¹ représente NR¹R² et R¹ et R² sont tous deux un isopropyle.

6. Procédé selon la revendication 3 ou 5 dans lequel R³ et R⁴ sont tous deux un hydrogène, R⁵ est -(CH₂)₆ et Y¹, Y² et Y³ sont tous un benzoyle ou un diméthoxytrityle.

7. Procédé selon l'une quelconque des revendications précédentes dans lequel X¹ représente NR¹R² et R¹ et R² sont tous deux un isopropyle, G est -(CH₂)₄, et soit R est un β-cyanoéthyle, soit Z a la formule : 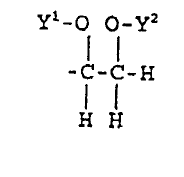
dans laquelle Y¹ et Y² sont tous deux un benzoyle ou Y¹ est un benzoyle et Y² est un diméthoxytrityle.

8. Procédé selon la revendication 1 ou 2 dans lequel Z représente un groupe diol adjacent à hydroxy protégé de formule : 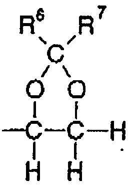
dans laquelle chaque R⁶ et R⁷ représentent individuellement un atome d'hydrogène, un groupe aryle en C₆-C₃₀ ou aryle substitué, alkyle en C₁-C₂₀ ou alkyle de moins de 30 atomes de carbone substitué par des groupes aromatiques ;
à la condition que R⁶ et R⁷ ne soient pas tous deux des atomes d'hydrogène.

9. Procédé selon la revendication 8 dans lequel chaque R⁶ et R⁷ représentent individuellement un atome d'hydrogène ou un phényle éventuellement substitué par un alkyle en C₁-C₈, un alkoxy en C₁-C₈, 1 à 4 atomes d'halogène chacun de nombre atomique compris entre 9 et 35, un nitro, ou un phényle, à la condition que R⁶ et R⁷ ne soient pas tous deux des atomes d'hydrogène.

10. Procédé selon la revendication 8 ou 9 dans lequel R⁶ représente un atome d'hydrogène et R⁷ représente un groupe phényle, p-butylphényle, p-méthoxyphényle, p-t-butylphényle ou biphényle.

11. Procédé selon la revendication 1 ou 2 dans lequel X¹ représente NR¹R² et R¹ et R² sont tous deux un isopropyle, G est -(CH₂)₆-, et Z a la formule :
-S-S-R⁵-O-Y³
dans laquelle R⁵ est -(CH₂)₆- et Y³ est un diméthoxytrityle.

12. Procédé selon l'une quelconque des revendications précédentes comprenant en outre l'étape consistant à éliminer les groupes protecteurs d'hydroxy pour laisser des groupes hydroxy libres.

13. Procédé selon la revendication 12 comprenant en outre l'étape consistant à oxyder Z pour former un groupe aldéhyde terminal sur l'oligonucléotide.

14. Procédé selon la revendication 11 comprenant en outre l'étape consistant à réduire Z pour former un groupe thiol terminal sur l'oligonucléotide.

15. Procédé selon la revendication 13 comprenant en outre l'étape consistant à conjuguer une molécule porteuse contenant un groupe amino avec ledit groupe aldéhyde terminal.

16. Procédé selon la revendication 14 comprenant en outre l'étape consistant à conjuguer une molécule porteuse contenant un groupe fonctionnel avec ledit groupe thiol terminal.

17. Procédé selon la revendication 15 ou 16 dans lequel la molécule porteuse est un polymère.

18. Procédé selon la revendication 17 dans lequel le polymère est un polymère d'acides aminés.

19. Composé de formule : 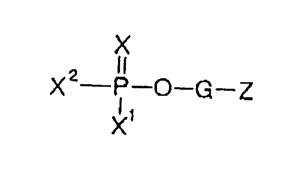 dans laquelle
X est :
(i) un atome d'oxygène ou de soufre lorsque X¹ représente O⁻, un méthyle ou -OCH₃ et X² représente un atome d'hydrogène ou RO-, dans laquelle R représente un groupe protecteur ;
(ii) non présent lorsque
(a) X¹ représente un atome de chlore et X² représente un méthyle ou RO- ; ou
(b) X² représente RO- et X¹ représente NR¹R² dans laquelle chaque R¹ et R² représentent individuellement un groupe alkyle en C₁-C₆, cycloalkyle en C₃-C₈ ou aryle en C₆-C₂₀ ou, lorsqu'ils sont attachés ensemble, forment avec l'atome d'azote une structure cyclique en C₄-C₇ avec 0 ou 1 atome d'oxygène ou de soufre ;
G représente un groupe hydrocarbylène en C₁-C₂₀ ; et
Z représente un groupe diol voisin à hydroxy protégé, lié à G par l'un des atomes de carbone du diol voisin ou un groupe disulfure lié à G par l'un des atomes de soufre du groupe disulfure ;
à la condition que G ait au moins 4 atomes de carbone lorsque Z représente ledit groupe disulfure.

20. Composé de formule : 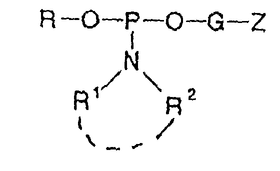
dans laquelle R représente un groupe méthyle ou un groupe protecteur sensible aux bases ;
chaque R¹ et R² représentent individuellement un groupe alkyle en C₁-C₆, cycloalkyle en C₃-C₈, ou aryle en C₆-C₂₀ ou, lorsqu'ils sont attachés ensemble, forment avec l'atome d'azote une structure cyclique en C₄-C₇ ayant 0 ou 1 atome d'oxygène ou de soufre ;
G représente un groupe hydrocarbylène en C₁-C₂₀ ; et
Z représente un groupe diol adjacent à hydroxy protégé lié à G par l'un des atomes de carbone du diol voisin ou un groupe disulfure lié à G par l'un des atomes de soufre du groupe disulfure ;
à la condition que G ait au moins 4 atomes de carbone lorsque Z représente ledit groupe disulfure.

21. Composé selon la revendication 19 ou 20 dans lequel Z a la formule : 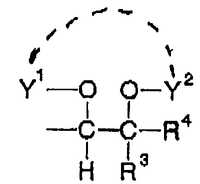
dans laquelle chaque R³ et R⁴ représentent individuellement un atome d'hydrogène, un groupe alkyle en C₁-C₂₀ ou aryle monocyclique en C₆-C₂₀, et chaque Y¹ et Y² représentent individuellement un groupe protecteur d'hydroxy ou sont attachés ensemble par un pont monoatomique pour former un groupe protecteur cyclique à cinq membres ;
ou Z représente
-S-S-R⁵-O-Y³
dans lequel R⁵ représente un groupe alkylène en C₁-C₂₀ ou un groupe arylène monocyclique en C₆-C₂₀ et Y³ est un groupe protecteur d'hydroxy.

22. Composé selon la revendication 21 dans lequel R⁵ représente un groupe alkylène en C₄-C₆, -OY³ est lié à l'atome ω du groupe alkylène et Y³ est un trityle.

23. Composé selon l'une quelconque des revendications 19 à 22 dans lequel X¹ représente NR¹R² et R¹ et R² sont tous deux un isopropyle.

24. Composé selon la revendication 21 ou 23 dans lequel R³ et R⁴ sont tous deux un hydrogène, R⁵ est -(CH₂)₆, et Y¹, Y² et Y³ sont tous un benzoyle ou un diméthoxytrityle.

25. Composé selon l'une quelconque des revendications 19 à 24 dans lequel X¹ représente NR¹R² et R¹ et R² sont tous deux un isopropyle, G est -(CH₂)₄, et soit R est un β-cyanoéthyle, soit Z a la formule : 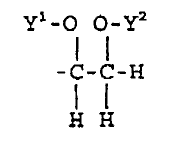
dans laquelle Y¹ et Y² sont tous deux un benzoyle ou Y¹ est un benzoyle et Y² est un diméthoxytrityle.

26. Procédé selon la revendication 19 ou 20 dans lequel Z représente un groupe diol voisin à hydroxy protégé de formule : 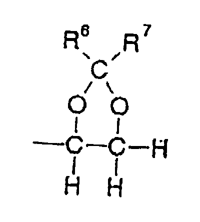
dans laquelle chaque R⁶ et R⁷ représentent individuellement un atome d'hydrogène, un groupe aryle en C₆-C₃₀ ou aryle substitué, alkyle en C₁-C₂₀ ou alkyle de moins de 30 atomes de carbone substitué par des groupes aromatiques ;
à la condition que R⁶ et R⁷ ne soient pas tous deux des atomes d'hydrogène.

27. Composé selon la revendication 26 dans lequel chaque R⁶ et R⁷ représentent individuellement un atome d'hydrogène ou un phényle éventuellement substitué par un alkyle en C₁-C₈, un alkoxy en C₁-C₈, 1 à 4 atomes d'halogène chacun de nombre atomique compris entre 9 et 35, un nitro, ou un phényle, à la condition que R⁶ et R⁷ ne soient pas tous deux des atomes d'hydrogène.

28. Composé selon la revendication 27 ou 28 dans lequel R⁶ représente un atome d'hydrogène et R⁷ représente un groupe phényle, p-butylphényle, p-méthoxyphényle, p-t-butylphényle ou biphényle.

29. Composé selon la revendication 19 ou 20 dans lequel X¹ représente NR¹R² et R¹ et R² sont tous deux un isopropyle, G est -(CH₂)₆-, et Z a la formule :
-S-S-R⁵-O-Y³
dans laquelle R⁵ est -(CH₂)₆- et Y³ est un diméthoxytrityle.

30. Oligonucléotide modifié en 5' de formule : 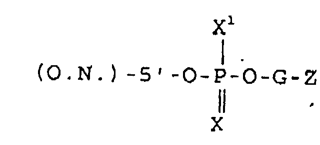
dans laquelle (O.N.) représente une chaîne oligonucléotidique ;
X est un atome d'oxygène ou de soufre ;
X¹ représente O⁻, un méthyle, -OCH₃ ou NR¹R² dans lequel chaque R¹ et R² représentent individuellement un atome d'hydrogène ou un groupe alkyle en C₁-C_{6,} cycloalkyle en C₃-C_{8,} ou aryle en C₆-C₂₀ ou, lorsqu'ils sont attachés ensemble, forment avec l'atome d'azote une structure cyclique en C₄-C₇ ne contenant aucun ou un seul atome d'oxygène ou de soufre ; G est un groupe hydroxycarbylène en C₁-C₂₀ ; et
Z est un groupe diol voisin à hydroxy protégé lié à G par l'un des atomes de carbone du diol voisin ou un groupe disulfure lié à G par l'un des atomes de soufre du groupe disulfure.

31. Oligonucléotide modifié en 5' selon la revendication 30 dans lequel Z a la formule : 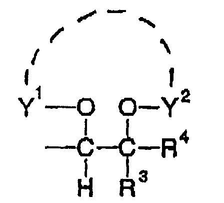
dans laquelle chaque R³ et R⁴ représentent individuellement un atome d'hydrogène, un groupe alkyle en C₄-C₂₀ ou aryle monocyclique en C₆-C₂₀ et chaque Y¹ et Y² représentent individuellement un groupe protecteur d'hydroxy ou sont attachés ensemble par un pont monoatomique pour former un groupe protecteur cyclique à cinq membres, ou
-S-S-R⁵-O-Y³
dans lequel R⁵ représente un groupe alkylène en C₁-C₂₀ ou un groupe arylène monocyclique en C₆-C₂₀ et Y³ est un groupe protecteur d'hydroxy.

32. Oligonucléotide modifié en 5' selon la revendication 31 dans lequel Y¹ et Y² sont éliminés pour laisser des groupes hydroxy libres.

33. Oligonucléotide modifié en 5' selon la revendication 31 dans lequel G est -(CH₂)₄-, et Z a la formule : 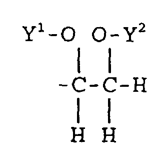
dans laquelle Y¹ et Y² sont tous deux un benzoyle ou Y¹ est un benzoyle et Y² est un diméthoxytrityle.

34. Oligonucléotide modifié en 5' selon la revendication 33 dans lequel Y¹ et Y² sont éliminés pour laisser des groupes hydroxy libres.

35. Oligonucléotide modifié en 5' selon la revendication 30 ou 31 dans lequel Z a la formule : 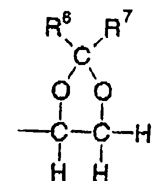
dans laquelle chaque R⁶ et R⁷ représentent individuellement un atome d'hydrogène, un aryle en C₆-C₃₀ ou un aryle substitué, un alkyle en C₁-C₂₀ ou un alkyle de moins de 30 atomes de carbone substitué par des groupes aromatiques ;
à la condition que R⁶ et R⁷ ne soient pas tous deux des atomes d'hydrogène.

36. Oligonucléotide modifié en 5' selon la revendication 35 dans lequel chaque R⁶ et R⁷ représentent individuellement un atome d'hydrogène ou un phényle éventuellement substitué par un alkyle en C₁-C₈, un alkoxy en C₁-C₈, 1 à 4 atomes d'halogène chacun de nombre atomique compris entre 9 et 35, un nitro, ou un phényle, à la condition que R⁶ et R⁷ ne soient pas tous deux des atomes d'hydrogène.

37. Oligonucléotide modifié en 5' selon la revendication 35 ou 36 dans lequel R⁶ est un atome d'hydrogène et R⁷ représente un groupe phényle, p-butylphényle, p-méthoxyphényle, p-t-butylphényle ou biphényle.

38. Oligonucléotide modifié en 5' selon la revendication 30 ou 31 dans lequel G est -(CH₂)₆- et Z a la formule :
-S-S-R⁵-O-Y³
dans laquelle R⁵ est -(CH₂)₆- et Y³ est un diméthoxytrityle.

39. Oligonucléotide modifié en 5' selon la revendication 32 ou 34 dans lequel Z a été oxydé pour former un groupe aldéhyde terminal sur l'oligonucléotide.

40. Oligonucléotide modifié en 5' selon la revendication 38 dans lequel Z a été réduit pour former un groupe thiol terminal sur l'oligonucléotide.

41. Conjugué d'une molécule porteuse contenant un groupe amino et d'un oligonucléotide modifié en 5' selon la revendication 39, dans lequel le conjugué est formé par réaction entre ledit groupe amino et ledit groupe aldéhyde terminal.

42. Conjugué d'une molécule porteuse ayant un groupe fonctionnel et d'un oligonucléotide modifié en 5' selon la revendication 40, dans lequel le conjugué est formé par réaction entre ledit groupe fonctionnel et ledit groupe thiol terminal.

43. Conjugué selon la revendication 41 ou 42 dans lequel la molécule porteuse est un polymère.

44. Conjugué selon la revendication 43 dans lequel le polymère est un polymère d'acides aminés.

45. Alcool partiellement protégé de formule : 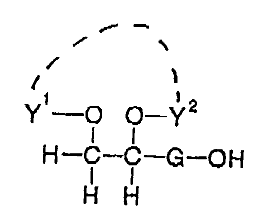
dans laquelle chaque Y¹ et Y² représentent individuellement un groupe protecteur d'hydroxy ou sont attachés ensemble par un pont monoatomique, autre qu'un groupe de pontage -C(=O)-, pour former un groupe protecteur cyclique à cinq membres et G représente un groupe hydroxycarbylène en C₂-C₂₀.

46. Alcool selon la revendication 45 dans lequel Y¹ et Y² sont tous deux un benzoyle et G est un butylène.

47. Alcool selon la revendication 45 dans lequel Y¹ est un diméthoxytrityle, Y² est un benzoyle et G est un butylène.

48. Alcool selon la revendication 45 dans lequel chaque Y¹ et Y² représentent individuellement : 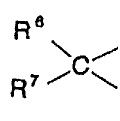
dans lequel chaque R⁶ et R⁷ représentent individuellement un atome d'hydrogène, un groupe aryle en C₆-C₃₀ ou aryle substitué, alkyle en C₁-C₂₀ ou alkyle de moins de 30 atomes de carbone substitué par des groupes aromatiques, à la condition que R⁶ et R⁷ ne soient pas tous deux des atomes d'hydrogène.

49. Disulfure de formule
Y³-O-G-S-S-G-OH
dans laquelle G représente un groupe hydrocarbylène en C₄-C₂₀ et Y³ représente un groupe protecteur d'hydroxyle.

50. Disulfure selon la revendication 49 dans lequel Y³ est un diméthoxytrityle et G est un hexylène.
